# EUROPEAN PATENT SPECIFICATION

(11) **EP 2 691 512 B1**
(45) Date of publication and mention of the grant of the patent: **01.05.2019**
(21) Application number: 12763669.4
(22) Date of filing: 28.03.2012
(51) Int. Cl.: C12N 5/077, C12N 5/0775, C12N 13/00, C12N 5/00

(54) **ENRICHED POPULATIONS OF CARDIOMYOCYTE LINEAGE CELLS FROM PLURIPOTENT STEM CELLS**
ANGEREICHERTE POPULATIONEN VON HERZMUSKELZELLEN AUS PLURIPOTENTEN STAMMZELLEN
POPULATIONS ENRICHIES DE CELLULES DE LIGNÉE CARDIOMYOCYTAIRE ISSUES DE CELLULES SOUCHES PLURIPOTENTES

(30) Priority: 29.03.2011 US 201161468952 P
(43) Date of publication of application: 05.02.2014
(73) Proprietor: Asterias Biotherapeutics, Inc., Fremont CA 94555 (US)
(72) Inventor: O'SULLIVAN, Christopher, San Mateo, CA 94404 (US); NISHIMOTO, Kevin, San Mateo, CA 94401 (US); REDDY, Anita, San Ramon, CA 94582 (US)
(74) Representative: Bassil, Nicholas Charles
(86) International application number: PCT/US2012/030799
(87) International publication number: WO 2012/135253

(56) References cited:
- WO-A1-2008/048931
- WO-A1-2009/017460
- WO-A1-2010/151782
- WO-A1-2012/024782
- US-A1- 2006 002 906
- US-A1- 2008 131 509
- US-A1- 2009 148 422
- US-A1- 2010 226 927
- US-A1- 2010 330 670
- US-B2- 7 364 863
- MICHAEL A LAFLAMME ET AL: "Cardiomyocytes derived from human embryonic stem cells in pro-survival factors enhance function of infarcted rat hearts", NATURE BIOTECHNOLOGY, vol. 25, no. 9, 1 September 2007 (2007-09-01), pages 1015-1024, XP55008206, ISSN: 1087-0156, DOI: 10.1038/nbt1327
- FLAVIUS C PASCUT ET AL: "Noninvasive Detection and Imaging of Molecular Markers in Live Cardiomyocytes Derived from Human Embryonic Stem Cells", BIOPHYSICAL JOURNAL, CELL PRESS, US, vol. 100, no. 1, 23 November 2010 (2010-11-23), pages 251-259, XP028153686, ISSN: 0006-3495, DOI: 10.1016/J.BPJ.2010.11.043 [retrieved on 2010-12-06]
- HONDA M ET AL: "N-cadherin is a useful marker for the progenitor of cardiomyocytes differentiated from mouse ES cells in serum-free condition", BIOCHEMICAL AND BIOPHYSICAL RESEARCH COMMUNICATIONS, ACADEMIC PRESS INC. ORLANDO, FL, US, vol. 351, no. 4, 29 December 2006 (2006-12-29), pages 877-882, XP024925771, ISSN: 0006-291X, DOI: 10.1016/J.BBRC.2006.10.126 [retrieved on 2006-12-29]
- CHUNHUI XU ET AL: "Efficient generation and cryopreservation of cardiomyocytes derived from human embryonic stem cells", REGENERATIVE MEDICINE, vol. 6, no. 1, 1 January 2011 (2011-01-01) , pages 53-66, XP55096219, ISSN: 1746-0751, DOI: 10.2217/rme.10.91

## Description

### FIELD OF THE INVENTION

The invention relates to a method of reducing the number of mesenchymal stem cells (MSC) in a mixed population of cells comprising cardiomyocyte lineage cells.

### BACKGROUND

Pluripotent stem cells have the ability to both proliferate in culture and, under appropriate growth conditions, differentiate into lineage restricted cell types representative of all three primary germ layers: endoderm, mesoderm and ectoderm (U.S. Patent Nos. 5,843,780; 6,200,806; 7,029,913; Shamblott et al., (1998) Proc. Natl. Acad. Sci. USA 95:13726; Takahashi et al., (2007) Cell 131(5):861; Yu et al., (2007) Science 318:5858). Defining appropriate growth conditions for particular lineage restricted cell types will provide virtually an unlimited supply of that cell type for use in research and therapeutic applications.

Protocols for differentiating primate pluripotent stem (pPS) cells into a variety of targeted cell types including oligodendrocytes, neuronal cells, cardiomyocytes, hematopoietic cells, pancreatic islet cells, hepatocytes, osteoblast and chondrocytes have been described (see, e.g., U.S. Patent Nos. 7,285,415; 6,833,269; 7,425,448; 7,452,718; 7,033,831; 7,326,572; 6,458,589; 6,506,574; 7,256,042; 7,473,555; U.S. Patent Publication Nos. 2005/0158855; 2004/0224403; 2005/0282272; 2005/0282274; 2006/0148077; U.S. Patent Application No. 12/412,183; PCT Publication No. WO 07/149182; WO 05/097980; Carpenter et al. (2001) Exp Neurology 172:383; Chadwick et al. (2003) Blood 102:906; Kierstad et al. (2005) J Neuroscience 25:4694); Laflamme et al. (2007) Nature Biotechnology 25:1015 Jiang et al. (2007) Stem Cells 25:1940.

Differentiation of pPS cells into a target phenotype cell may result in production of a mixed population of cells comprising the targeted phenotype as well as various extraneous phenotypes. Certain extraneous phenotypes that retain the pluripotent potential of undifferentiated embryonic stem cells may form teratomas when administered to a subject, see, e.g., Thomson 1998 Science 282:1145. Other extraneous phenotypes may interfere with the efficacy of the target phenotype merely by diluting the number of cells of the targeted phenotype thereby reducing the overall efficacy of the cell preparation. Accordingly, there is a need to reduce the number of cells having an extraneous phenotype found in a population of cells comprising the targeted differentiated progeny of pPS cells. There is an additional need for populations of differentiated progeny of pPS cells that have a minimal number of cells of an extraneous phenotype for use in therapeutic, diagnostic and/or research applications.

### SUMMARY OF THE INVENTION

The present disclosure relates to the discovery that during the course of differentiation of primate pluripotent stem cells to a targeted phenotype, populations of extraneous phenotypes may arise and that by depleting cells expressing molecules found on or in these extraneous phenotypic cells the targeted phenotype may be enriched and the extraneous phenotype may be depleted relative to the starting population. By enriching for the target phenotype the invention provides improved populations of these cells and thus provides for better therapeutic agents, better research agents and better screening agents.

According to the invention, there is provided a method of reducing the number of mesenchymal stem cells (MSC) in a mixed population of cells comprising cardiomyocyte lineage cells which are the in vitro differentiated progeny of pPS cells, the method comprising: a) contacting the mixed cell population with one or more ligands that bind to a MSC marker chosen from CD90, CD73, CD140b, CD10, and CD44 ; and b) removing the ligand bound cells of a) thereby reducing the number of MSC from the mixed population of cells comprising cardiomyocyte lineage cells. The one or more ligands that bind to a MSC marker can be chosen from CD90, CD73, CD140b, CD10 and CD44 is one or more antibodies. The one or more antibodies can be a monoclonal antibody. The one or more antibodies can be a polyclonal antibody. The one or more antibodies can be an antibody that binds to CD90.

According to the invention, the removal of the ligand bound cells may comprise contacting the cell population of a) with an external force. The external force can be a magnetic field.

According to the invention, the ligand may be bound to a solid support. The ligand can be directly bound to the solid support. The ligand can be indirectly bound to the solid support. The solid support can be a bead. The bead can be a magnetic bead.

According to the invention, the cardiomyocyte lineage cells may express one or more markers chosen from CD106, cardiac troponin I (cTnl); cardiac troponin T (cTnT); Nkx2.5; ANF, myosin heavy chain (MHC); titin; tropomyosin; α sarcomeric actinin; desmin; GATA-4; MEF 2A; MEF 2B; MEF 2C: MEF 2D; N-cadherin; connexin 43; β1 adrenoreceptor (β1 AR); creatine kinase MB (CK MB); myoglobin; α cardiac actin. The obtaining step can comprise differentiating in vitro an aliquot of a line of pPS cells into cardiomyocyte lineage cells. The ligand can be conjugated to a detectable substance. The detectable substance can be a dye.

According to the invention, removing the ligand bound cells may comprise sorting the ligand bound cells using a flow cytometer.

Disclosed herein are improved populations of cells which are the differentiated progeny of primate pluripotent stem cells such as cardiomyocyte lineage cells, hematopoietic lineage cells. The improved populations are depleted of one or more extraneous phenotypic cells such as non-cardiomyocyte lineage cells.

Surprisingly, it has been found that CD90 is a marker of extraneous phenotypic cells found in various populations of cells which are the in vitro differentiated progeny of pPS cells such as cardiomyocyte lineage cells, hematopoietic lineage. By depleting cells expressing CD90 more suitable populations of targeted phenotypic cells, e.g., cardiomyocyte lineage cells, hematopoietic lineage cells are obtained. CD90 may be a marker for mesenchymal stem cells which have the ability to differentiate in epithelial cells, chondrocytes and osteoblasts. The removal of extraneous phenotypic cells may substantially reduce the number of MSC or there differentiated progeny occurring in cell populations comprising the in vitro differentiated progeny of pPS cells, such as cardiomyocyte lineage cells.

Disclosed herein is a population of cells enriched for cardiomyocyte lineage cells that are the in vitro differentiated progeny of pPS cells. For example the population of cardiomyocyte lineage cells may be enriched by depleting at least
one extraneous phenotypic cell from the population. Thus the population of cardiomyocyte lineage cells may be enriched relative to a starting population of cells comprising the cardiomyocyte lineage cells that has not been depleted of at least one extraneous phenotypic cell.

As disclosed herein the population of cells enriched for cardiomyocyte lineage is essentially free of extraneous phenotypic cell types, wherein the cardiomyocyte lineage cell is the in vitro differentiated progeny of pPS cells.

Also disclosed herein is a population of cells enriched for cardiomyocyte lineage cells comprising a population of cells comprising cardiomyocyte lineage cells, wherein at least one cell expressing a marker not found on a cardiomyocyte lineage cell has been depleted from the population of cells and, wherein the cardiomyocyte lineage cell is the in vitro differentiated progeny of pPS cells.

. Also disclosed herein is a population of cells comprising cardiomyocyte lineage cells depleted of at least one cell expressing CD90, wherein the cardiomyocyte lineage cell is the in vitro differentiated progeny of pPS cells.

Also disclosed herein is a population of cells comprising cardiomyocyte lineage cells depleted of at least one mesenchymal stem cell (MSC), wherein the cardiomyocyte lineage cell is the in vitro differentiated progeny of pPS cells.

Also disclosed herein is a composition comprising a population of cells comprising the in vitro differentiated progeny of pPS cells, wherein the composition comprises CD90+ cells and an exogenously added ligand that binds to CD90. The population of cells may comprise cardiomyocyte lineage cells.

Also disclosed herein is a composition comprising a population of cells comprising the in vitro differentiated progeny of pPS cells, wherein the composition comprises mesenchymal stem cells (MSC) and an exogenously added ligand that binds to a MSC. The population of cells may comprise e.g., cardiomyocyte lineage cells, hematopoietic lineage cells.

Also disclosed herein is a method of enriching a population of cells which are the in vitro differentiated progeny of pPS cells such as cardiomyocyte lineage cells, hematopoietic lineage cells, comprising a) obtaining a population of cells comprising cardiomyocyte lineage cells which are the in vitro differentiated progeny of pPS cells; b) contacting the cell population of a) with one or more ligands that bind to a marker found on an MSC and c) removing the ligand bound cells of b) thereby obtaining a population of cells that is enriched for cardiomyocyte lineage cells. Removing the ligand bound cell can include physically separating the ligand bound cell from the rest of cell population.

Also disclosed herein is a method of enriching a population of cells, which are the in vitro differentiated progeny of pPS cells, e.g., cardiomyocyte lineage cells, hematopoietic lineage cells, comprising a) obtaining a population of cells comprising cardiomyocyte lineage cells which are the in vitro differentiated progeny of pPS cells; b) contacting the cell population of a) with one or more ligands that bind to CD90 and c) removing the ligand bound cells of b) thereby obtaining a population of cells that is enriched for cardiomyocyte lineage cells. Removing the ligand bound cell can include physically separating the ligand bound cell from the rest of cell population.

Also disclosed herein is a method of producing an enriched target cell population comprising a) obtaining a cell population that is the in vitro differentiated progeny of pPS cells; contacting the population of a) with a ligand that binds to CD90 and c) removing at least one ligand bound cell from b) thereby producing an enriched target cell population. Suitable target cell populations include cardiomyocyte lineage cells, hematopoietic lineage cells.

Removing the ligand bound cell as referred to in the methods described *infra* may include physically separating the ligand bound cell from the rest of cell population. Removing the ligand bound cell may also include killing the ligand bound cell. For example an agent such as toxin that binds to the ligand bound to the cell may be used. As described herein, the ligand may be antibody and complement may be used as agent that lyses the cell and thus removes it from the population of cells

Also described herein is a method of transplanting a population of cells comprising cardiomyocyte lineage cells into a subject comprising a) obtaining a population of cells comprising cardiomyocyte lineage cells, wherein the population of cells has been depleted of a least one MSC; and b) administering the population of cells from a) to the subject. The cardiomyocyte lineage cells may be the in vitro differentiated progeny of a line of pPS cells.

Also described herein is a method of transplanting a population of cells comprising cardiomyocyte lineage cells into a subject comprising a) obtaining a population of cells comprising cardiomyocyte lineage cells, wherein the population of cells has been depleted of a least one CD90+ cell; and b) administering the population of cells from a) to the subject. The cardiomyocyte lineage cells may be the in vitro differentiated progeny of a line of pPS cells.

Also described herein is a method of treating a subject in need of cellular therapy comprising a) obtaining a population of cells comprising cardiomyocyte lineage cells, wherein the population of cells has been depleted of a least one MSC and b) administering the population of cells from a) to the subject. The cardiomyocyte lineage cells may be the in vitro differentiated progeny of a line of pPS cells.

Also described herein is a method of treating a subject in need of cellular therapy comprising a) obtaining a population of cells comprising cardiomyocyte lineage cells, wherein the population of cells has been depleted of a least one CD90+ cell and b) administering the population of cells from a) to the subject. The cardiomyocyte lineage cells may be the in vitro differentiated progeny of a line of pPS cells.

Also described herein is the use of a population of cells for treating a subject in need of cellular therapy comprising cardiomyocyte lineage cells, wherein the population of cells has been depleted of a least one MSC. The cardiomyocyte lineage cells may be the in vitro differentiated progeny of a line of pPS cells.

Also described herein is the use of a population of cells for treating a subject in need of cellular therapy comprising cardiomyocyte lineage cells, wherein the population of cells has been depleted of a least one CD90+ cell. The cardiomyocyte lineage cells may be the in vitro differentiated progeny of a line of pPS cells.

Also described herein is a kit for depleting cells having an extraneous phenotype from a population of cells that includes cardiomyocyte lineage cells comprising a) a ligand for one or more molecules expressed by MSC; b) and a population of cardiomyocyte lineage cells which are the in vitro differentiated progeny of a pPS cell; and c) one or more containers.

Also described herein is a kit for depleting cells having an extraneous phenotype from a population of cells comprising a) a ligand for CD90; b) and a population of cardiomyocyte lineage cells which are the in vitro differentiated progeny of a pPS cell; and c) one or more containers.

Also described herein is a kit for depleting cells having an extraneous phenotype from a mixed population of cells comprising a) a ligand for one or more molecules expressed by MSC; b) instructions for depleting MSCs from the mixed population of cells, wherein the mixed population of cells comprises cardiomyocyte lineage cells that are the progeny of a pPS cell; and c) one or more containers.

Also described herein is a kit for depleting cells having an extraneous phenotype from a mixed population of cells comprising a) a ligand for CD90; b) instructions for depleting CD90+ from the mixed population of cells, wherein the mixed population of cells comprises cardiomyocyte lineage cells that are the progeny of a pPS cell; and c) one or more containers.

### DESCRIPTION OF THE FIGURES

Figure 1A shows bright field micrographs of MSC in cell culture.
Figure 1B shows flow cytometric analysis of MSC.
Figure 1C shows detection of calcium deposits in MSC differentiated in vitro using osteoblasts differentiation media.
Figure 2 shows flow cytometric analysis of 3 lots of cardiomyocyte lineage cells depleted of CD90+ cells.
Figure 3A and 3B show bright field micrographs of colony forming units (CFU) in three cell populations: 1) a non-depleted cardiomyocyte lineage cell prep; 2) CD90 depleted fraction; and 3) a CD90 enriched fraction.
Figure 3C shows calcium deposits in the same fractions shown in Figures 3A and 3B after differentiation to osteoblasts as described in Example 1. The CD 90 depleted fraction had few if any calcium deposits.
Figure 4A shows flow cytometric analysis of pre and post CD90 depletion of a population of cells comprising cardiomyocyte lineage cells which are the in vitro differentiated progeny of a line of pPS cells.
Figure 4B shows flow cytometric analysis of the same populations analyzed in Figure 4A, but stained here for cardiomyocyte marker TnI.
Figure 4C is a graph showing the reduction of CFUs in CD90 depleted populations compared to pre- CD90 depleted populations.
Figure 5A shows the result of a cell sorting experiment using Fluorescent Activated Cell Sorting (FACS). A comparison of the percentage of CD90+ cells in the pre-sorted population and the post sorted (depleted) CD90 population is provided.
Figure 5B shows the same two populations shown in Figure 5A stained here for cardiomyocyte marker TnI. The CD90 depleted fraction is enriched for TnI positive cells.
Figure 5C is a graph showing the number of CFUs present in the pre- CD90 depleted population compared to the number of CFUs present in the post CD90 depleted population.
Figure 6 shows flow cytometric analysis of a preparation of cardiomyocyte lineage cells. Within the preparation of cells are cells expressing markers found on mesenchymal stem cells (MSC).

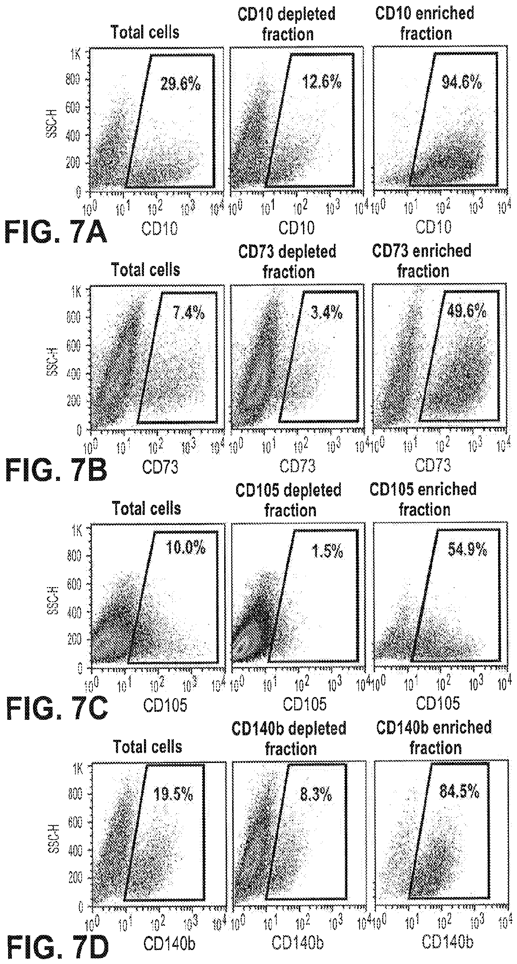

Figure 7 shows flow cytometric analysis of a population of cardiomyocyte lineage cells depleted for MSC markers a) CD10; b) CD73; c) CD105 d) CD140b.
Figure 8 is a graph showing that depletion of cells expressing MSC markers CD10, CD73 CD105 and CD140b from a population of cardiomyocyte lineage cells reduces the number of colony forming units from the population of cardiomyocyte lineage cells.
Figure 9 Shows micrographs demonstrating that depletion of CD105 and CD73 from a population of cardiomyocyte lineage cells reduce the number of cells staining positive for calcium deposits. Calcium staining is indicative of the presence of MSC progeny cell types and thus demonstrates that sorting for CD140b and CD10 reduces the number of MSC in the cardiomyocyte lineage cell population.
Figure 10a shows flow cytometric analysis of a population of cardiomyocyte lineage cells sorted for the presence of MSC markers Cd140b and CD10.
Figure 10b is a graph showing that depletion of CD140b and CD10 reduces the number of colony forming units from a population of cardiomyocyte lineage cells indicating a reduction in the number of MSCs in the population of cardiomyocyte lineage cells.
Figure 10c are micrographs showing that fractions sorted for the absence of CD 140b and CD10 had fewer cells staining positive for calcium compared with the starting pre-sorted population. Calcium staining is indicative of the presence of MSC progeny cell types and thus demonstrates that sorting for CD140b and CD10 reduces the number of MSC in the cardiomyocyte lineage cell population.
Figure 11 shows flow cytometric analysis of a population of cardiomyocyte lineage cells expressing CD106.
Figure 12 shows flow cytometric analysis of a population of cardiomyocyte lineage cells co-labeled with antibodies to CD106 and 1) antibodies to cardiac troponin I (left panel) and 2) α-actinin (right panel).
Figure 13 shows flow cytometric analysis of a population of cardiomyocyte lineage cells. The majority of CD 106 cells are CD90-.
Figure 14 shows flow cytometric analysis demonstrating that depletion of CD90+ cells from a population of cells comprising cardiomyocyte lineage cells enriches for CD106+ cells.

### DEFINITIONS

**"About,"** as used herein to refer to an amount or a value means + or - 5% of stated amount or value.
**"Antibody,"** as used herein refers to an immunoglobulin or a part thereof, and encompasses any polypeptide comprising an antigen-binding site regardless of the source, method of production, or other characteristics. The term includes for example, polyclonal, monoclonal, monospecific, polyspecific, humanized, single-chain, chimeric, synthetic, recombinant, hybrid, mutated, and CDR-grafted antibodies. A part of an antibody can include any fragment which can bind antigen, for example, an Fab, F(ab')₂, Fv, scFv as long as the fragment may be conjugated to or binds to another molecule which is not the antigen.
**"Cardiomyocyte lineage cells,"** as used herein includes mature cardiomyocytes (a cell that possesses the morphology and the functional capability of a cardiomyocyte isolated from either a juvenile or adult primate) and/or cardiomyocyte precursors (cells which express one or more markers expressed on, in, or by cardiomyocytes and/or have one or more morphological or functional attributes associated with cardiomyocytes and which may differentiate into mature cardiomyocyte either in vitro or when implanted into a subject).
**"Cell culture,"** as used herein, refers to a plurality of cells grown in vitro over time. The cell culture may originate from a plurality of pPS cells or from a single pPS cell and may include all of the progeny of the originating cell or cells, regardless of 1) the number of passages or divisions the cell culture undergoes over the lifetime of the culture; and 2) any changes in phenotype to one or more cells within the culture over the lifetime of the culture. Thus, as used herein, a cell culture begins with the initial seeding of one or more suitable vessels with at least one pPS cell and ends when the last surviving progeny of the original founder(s) is harvested or dies. Seeding of one or more additional culture vessels with progeny of the original founder cells is also considered to be a part of the original cell culture.
**"CD90,"** as used herein, refers to a 25-37kD N-glycosylated protein that is a member of the immunoglobulin family. It is expressed as a cell surface protein anchored to the cell membrane by a glycophosphatidylinositol (GPI) tail. CD90 is a marker for mesenchymal stem cells and hematopoietic stem cells. It is also expressed on CD34+ prothymocytes and neurons. It has been referred to in the literature as Thymocyte differentiation antigen 1 (Thy-1). Thus CD90 and Thy-1 may be used interchangeably to refer to the same molecule.
**"Depleted,"** as used herein, refers to an act by which an extraneous phenotype contained within a mixed population of cells has been decreased in number relative to other phenotypic cell types within the population as a result of an act initiated by the human hand. Included are methods where the extraneous phenotype is physically segregated from the other cells in the population, as for example by immuno-precipitating the extraneous phenotypic cells. Also included are methods where the extraneous phenotypic cells are removed chemically from the rest of the population, e.g., where the extraneous phenotypic cell is specifically targeted with a chemical agent such as a toxin, complement and the like. Typically the depletion is measured by comparing the number of cells bearing the extraneous phenotype before and after the act initiated by the human hand whereby any decrease in the relative number of cells in the population bearing the extraneous phenotype as a result of the act initiated by the human hand is considered depletion. Depletion of an extraneous phenotype from a mixed population of cells may result in the enrichment of a target cell population within the mixed population of cells.
**"Enriched,"** as use herein, refers to an act initiated by the human hand by which a target phenotype contained within a mixed population of cells has increased in number relative to other phenotypic cell types within the population. Typically the enrichment is measured by comparing the number of target phenotypic cells before and after the act initiated by the human hand whereby any increase in the relative number of the targeted cell population as a result of the act initiated by the human hand is considered enrichment.
**"Embryoid bodies,"** as used herein, refers to heterogeneous clusters comprising undifferentiated, differentiated and partly differentiated cells that appear when primate pluripotent stem cells are allowed to differentiate in a non-specific fashion in suspension cultures or aggregates.
As used herein, **"reference embryonic stem cell" (ES)** refers to pluripotent stem cells that are 1) derived from a blastocyst before substantial differentiation of the cells into the three germ layers or 2) alternatively obtained from an established cell line which is maintained in vitro over multiple passages. Except where explicitly required otherwise, the term includes primary tissue and established lines that bear phenotypic characteristics of ES cells, and progeny of such lines that have the pluripotent phenotype. The reference ES cells may be human ES cells (hES). Prototype reference "human Embryonic Stem cells" (hES cells) are described by Thomson et al. (Science 282:1145, 1998; U.S. Patent 6,200,806) and may be obtained from any one of a number of established stem cell banks such as the UK Stem Cell Bank (Hertfordshire, England) and the National Stem Cell Bank (Madison, WI).
**"Essentially free,"** as used herein, to refers to a cell population that contains less than 1% of an extraneous phenotype.
**"Extraneous phenotype,"** as used herein, refers to one or more cell types contained within a mixed population of cells that are undesirable. Thus in a mixed population of cells comprising a target phenotypic cell population, (such as cardiomyocyte lineage cells) any cell having a phenotype that differs from the target population may be considered extraneous. Additionally any cell that expresses one or more markers not found expressed by, on or in the target cell population may be considered an extraneous phenotype. Extraneous phenotypic cells may be targeted for depletion. An example of an extraneous phenotypic cell in a population of cardiomyocyte lineage cells is a cell expressing CD90.
**Hematopoietic lineage cells,** as used herein, refers to cells differentiated in vitro from pPS cells and/or their progeny and may include one or more of the following: hemangioblasts, hematopoietic stem cells, common lymphoid progenitor cells, lymphocytes, common myeloid progenitor cells (CMP), granulomonocytic progenitor cells (GMP), monocytes, macrophages, immature dendritic cell and mature dendritic cell. Markers for hematopoietic lineage cells may include one or more of the following: CD34+, CD59+, Thy1/CD90+, CD38^{lo/-}, C-kit/CD117^{- /lo}, CD13, CD34, IL-3Rα (CD123), CD45RA, CD14, CD45^{hi}, CD11a, CD11b, CD15, CD11c^{hi}, MHC I, MHC II^{lo}, CD205⁻, and CD83.
**"In vitro differentiated progeny,"** as used herein, refers to cells that have been differentiated from pPS cells in vitro. Differentiation of pPS cells may be achieved by altering the conditions which maintain the pluripotency of the pPS cells. Altering the conditions that maintain pluripotency may include the addition of one or more growth factors, cytokines, morphogens, or the like, that are added by the human hand to a culture of pPS cells or to a culture of pPS cells that have already begun to differentiate down one or more specific pathways. Additional suitable agents that may be useful in differentiating pPS include micro RNA molecules, siRNA molecules, and small molecules such as IDE1, IDE2, -(-)Indolactan and Stauplimide. (See, Borowick et al. (2009) Cell Stem Cell 4:348; Chen et al. (2009) Nature Chem Biol 4:258; Zhu (2009) Cell Stem Cell 4:416). Altering the conditions that maintain pluripotency may also include the removal of one or more growth factors and/or mitogens from the pPS culture such that the pPS cells begin to differentiate. As an example FGF such as βFGF may be removed from the culture of pPS cells. Additionally feeder cells, if present may be removed from the culture of pPS cells to initiate differentiation. Media conditioned by feeder cells may be removed and substituted with a non-conditioned media to initiate differentiation of pPS cells. Cells that have been differentiated from embryoid bodies are included in the definition provided. In some instances one or more growth factors, cytokines, morphogens or the like have been added to the culture before or after formation of the embryoid body in order to facilitate the differentiation down a particular pathway to obtain a target phenotype.
**"Ligand,"** as used herein, refers to a first molecule that specifically interacts with a second molecule to form a chemical bond. Typically, the bond will be a non-covalent bond such as an ionic interaction, a hydrogen bond or an interaction mediated by Van der Waals forces. Examples of ligand pairs (i.e. the first and second molecule referred to above) include an epitope and an antibody that binds specifically to that epitope; and a substrate that specifically binds an enzyme. The dissociation constant (K_{d}) between the two molecules is one measure of the affinity between a ligand and its specific binding partner. Specific interactions are typically characterized by relatively small K_{d}. An example of a ligand pair that bind each other with very high affinity (very small K_{d}) is biotin/ avidin. In contrast, non-specific binding between two molecules may involve the formation of chemical bonds based on charge and hydrophobicity, however, unlike specific binding the interactions are not based on the exact structure of the two molecules and are typically characterized by a large K_{d}, e.g., on the order of 10⁻⁴.
**"Marker,"** as used herein, refers to a molecule expressed either as a nucleic acid and/or as a protein, and/or a lipid, and/or a carbohydrate, and/or a combination of 2 or more of the preceding molecule types in, on or by a cell and which may be detected by any means known in the art. Markers are useful as a means of comparing and/or distinguishing one cell from another, e.g., a cell that expresses a particular molecule may be distinguished from one that does not. Markers may be detected using any method known in the art, for example immunocytochemistry, immunohistochemistry, FACS, western blot, ELISA, or qPCR may be used
**"Mesenchymal stem cells (MSC),"** as used herein, refers to multipotent stem cells having the ability to both proliferate in a multipotent state and the ability to differentiate into certain cell types such as osteoblasts, chondrocytes, adipocytes and other cell types (see, e.g. Alhadlaq and Mao (2004) Stem Cells Dev. 13:436; Kuroda et al. (2010) PNAS 107:8639. MSCs are derived from adult tissue and do not have the ability to form teratomas in the SKID mouse model, Kuroda et al. (2010) PNAS 107:8639. Thus MSCs are more developmentally advanced relative to pPS cells. While pPS cells have the ability to differentiate into any cell in the body and hence are termed "pluripotent," MSCs are generally believed to have a more limited capacity to differentiate and thus are termed "multipotent." Morphologically, MSCs are characterized by a small cell body, a large round nucleus and a prominent nucleolus and few long thin processes. MSCs may express CD90 and typically have the ability to form colony forming units (CFU) when cultured in vitro. Other markers expressed by MSCs include CD44, CD140b, CD10, CD73, CD105 and Stro-1. Sources of MSC in humans include bone marrow (adult), fetal liver, fetal blood, amniotic fluid, post natal peripheral blood and cord blood.
**"Mixed population of cells,"** as used herein, refers to an in vitro culture of cells comprised of cells having more than one phenotype and/or an in vitro culture of cells wherein at least one cell in the culture expresses at least one marker not found on at least one other cell in that culture.
As used herein, **"primate pluripotent stem cells"(pPS)** refers to cells that may be derived from any source and that are capable, under appropriate conditions, of producing primate progeny of different cell types that are derivatives of all of the 3 germinal layers (endoderm, mesoderm, and ectoderm). pPS cells may have the ability to form a teratoma in 8-12 week old SCID mice and/or the ability to form identifiable cells of all three germ layers in tissue culture. Included in the definition of primate pluripotent stem cells are
   reference human embryonic stem (hES) cells, (see, e.g., Thomson et al. (1998) Science 282:1145.);
   embryonic stem cells from other primates, such as Rhesus stem cells (see, e.g., Thomson et al., (1995) Proc. Natl. Acad. Sci. USA 92:7844), marmoset stem cells (see, e.g., (1996) Thomson et al., Biol. Reprod. 55:254,),
   as well as induced pluripotent stem cells (see, e.g. Yu et al., (2007) Science 318:5858); Takahashi et al., (2007) Cell 131(5):861)and pluripotent cells derived from adult human spermatagonial stem cells, Renninger, et al. (2008) Nature 20:456). The pPS cells may be established as cell lines, thus providing a continual source of pPS cells. It is contemplated that any of the embodiments of the invention described herein may be practiced by substituting one or more of the following sub-groupings of pPS cells for pPS cells: human embryonic stem cells, human embryonic germ cells, rhesus stem cells, marmoset stem cells, nuclear transfer stem cells and/or induced pluripotent stem cells.
**"Subject,"** as used herein, refers to any mammal, including, but not limited to, a human, a non-human primate, a pig, a horse, a cow, a dog, a cat, a rabbit, a guinea pig, a rat, and a mouse.
As used herein, **"undifferentiated primate pluripotent stem cells"** refers to a cell culture where a substantial proportion of primate pluripotent stem cells and their derivatives in the population display morphological characteristics of undifferentiated cells and maintain the capacity to give rise to at least one cell type from each of the three germ layers: endoderm, mesoderm and ectoderm. It is understood that colonies of undifferentiated cells within the population may be surrounded by neighboring cells that are partly differentiated.
**Treat, treatment, treating,** as used herein, means any of the following: the reduction in severity of a disease or condition; the reduction in the duration of a disease course; the amelioration of one or more symptoms associated with a disease or condition; the provision of beneficial effects to a subject with a disease or condition, without necessarily curing the disease or condition, the prophylaxis of one or more symptoms associated with a disease or condition.

### DETAILED DESCRIPTION OF THE INVENTION

The present disclosure relates, at least in part, to the discovery that extraneous phenotypes may sometimes exist in cell cultures comprising the in vitro differentiated progeny of pPS cells. Typically these extraneous phenotypes are rare and present at low levels. Nonetheless limiting the presence of these extraneous phenotypes would be useful. For example a population of cells comprising cardiomyocyte lineage cells that have been differentiated in vitro from a line of pPS cells, such as human reference embryonic stem (hES) cell may further comprise extraneous phenotypic cells. In some embodiments the extraneous phenotypic cells may be MSCs. In some embodiments the extraneous phenotypic cells may be CD90+. By depleting these extraneous phenotypes from the population the invention provides for an enriched population of cardiomyocyte lineage cells relative to the pre-depleted population of cells. Enriched populations of cardiomyocyte lineage cells according to the invention may be used for a variety of purposes including uses as therapeutic agents, research agents and agents in drug and toxicity screens.

### Methods of Reducing Extraneous Phenotypic Cells from a Mixed Population of Cells

Described herein is a method of reducing the number of extraneous phenotypic cells from a mixed population of cells comprising contacting the mixed population of cells with one or more ligands that bind to the cells having the extraneous phenotype and then removing, e.g., separating the extraneous phenotypic cells from the population. The mixed population of cells may comprise the in vitro differentiated progeny of pPS cells. In certain instances the mixed population of cells may comprise cardiomyocyte lineage cells that are the in vitro progeny of a line of pPS cells. In some instances the extraneous phenotype cell is an MSC. Thus in some instances the one or more ligands that bind to the extraneous phenotype cells may include one or more ligands that bind to an MSC. In some instances , the extraneous phenotype cell is a CD90+ cell. Thus in certain instances the one or more ligands that bind to the extraneous phenotype cells may include one or more ligands that bind to CD90. Suitable ligands may include an antibody. For example the antibody may be a monoclonal antibody or a polyclonal antibody.

In some instances a direct method of reducing the number of extraneous phenotype cells in a mixed population of cells may be used. The direct method may comprise binding one or more ligands to the extraneous phenotype cell directly. That is the one or more ligands will contact and bind to the extraneous phenotype cell without any intervening molecule. For example an antibody that binds directly to a molecule expressed on the cell surface of the extraneous phenotype cell may be used. A suitable ligand includes an antibody to CD90. In certain embodiments the antibody may be conjugated. For example the antibody may be conjugated to a dye, thus allowing separation of the bound cell by flow cytometry. As another example the antibody may be conjugated to a solid support (as described infra). Thus in one instance the direct method may be practiced using an antibody such as an anti-CD90 antibody conjugated to a bead, such as a magnetic bead. Accordingly a ligand may be directly bound to a molecule expressed by an extraneous phenotypic cell and then the bound cell may be removed, e.g., separated from the rest of the cell population.

In other instances the disclosure provides an indirect method of reducing the number of extraneous phenotype cells in a mixed population of cells. The indirect method may include a) contacting the population of cells first with one or more ligands that bind to a molecule expressed on the cell surface of the extraneous phenotype cell and b) contacting the cell population of a) with one or more ligands that bind to one or more of the ligands used in step a). The bound cells may then be removed, e.g. separated from the rest of the population of cells. In some instances the one or more ligands used in step a) may include an antibody to CD90. In some instances the one or more ligands in step b) may include an antibody that binds to the antibody used in step a). For example the antibody in step b) may specifically bind to the antibody isotype used in step a). In certain instances the antibody in step b) may be conjugated. For example the antibody may be conjugated to a dye, thus allowing separation of the bound cell by flow cytometry. As another example the antibody used in step b) may be conjugated to a solid support (as described infra). Thus in one instance the indirect method may be practiced using an antibody conjugated to a bead, such as a magnetic bead that specifically binds to an anti-CD90 antibody used in step a).

The mixed cell population may be contacted with ligand in a suitable buffer. Suitable buffers may include phosphate buffered saline (PBS) or any commercially available cell culture media that facilitates survival of the mixed population of cells. In certain embodiments the ligand may be provided in a buffer comprising PBS + 0.5% BSA + 2 mM EDTA. Typically the buffer will be an isotonic buffer and have a pH ranging from about 6.8 to about 7.7, from about 6.9 to about 7.6; from about 7.0 to about 7.5. In some instances the pH of the buffer will be physiological pH.

The method is adaptable over a wide range of sample volumes ranging from sample sizes obtainable from a single well of a microwell plate to a sample obtained from a scale-up bioreactor. A suitable volume for contacting the mixed cell population can range from about 50 µL to about 10,000 liters. In some instances a suitable volume for contacting the mixture of cells is about 100 µL, about 200 µL) about 500 µL, about 1 mL, about 10 mL, about 50 mL, about 100 mL, about 1 liter, about 10 liters, about 50 liters, about 100 liters, about 1000 liters, about 2000 liters, about 5000 liters, about 8,000 liters, about 10,000 liters or more.

Any suitable ratio of ligands to cell number comprising the mixed cell population may be used. For example a suitable ratio of ligand to cell number may be about 1:1; about 10:1; about 100:1; about 1000:1; about 10,000:1; about 10⁵:1; about 10⁶:1; about 10⁷:1; about 10⁸:1; about 10⁹:1; about 10¹⁰:1.

In some instances the amount of ligand used to contact the extraneous phenotype cell may be based on weight per unit volume. Thus in some instances the amount of ligand used to deplete an extraneous phenotype may range from about 0.0001 µg/µl to about 10µg/µl; from about 0.001µg/µl to about 1 µg/µl; from about 0.01 µg/µl to about 0.1µg/µl. In certain instances the concentration of ligand used to deplete an extraneous phenotype from a population of cells differentiated in vitro from a line of pPS cells may be about 0.4µg/µl

In some instances the ligand may be linked to a solid support such as a bead as described infra. A plurality of ligand linked beads may be used to reduce the number of extraneous phenotypic cells present in a mixed population of cells. For example, a suitable number of beads may be about 1 bead per cell; about 5 beads per cell; about 10 beads per cell; about 15 beads per cell; about 20 beads per cell; about 25 beads per cell; about 30 beads per cell; about 35 beads per cell; about 40 beads per cell; about 45 beads per cell; about 50 beads per cell; about 60 beads per cell; about 70 beads per cell; about 80 beads per cell; about 90 beads per cell; about 100 beads per cell. To each bead in turn may be linked to at least 10 ligands; at least 100 ligands; at least 1000 ligands; at least 10,000 ligands; at least 10⁵ ligands; at least 10⁶ ligands; at least 10⁷ ligands; at least 10⁸ ligands; at least 10⁹ ligands; at least 10¹⁰ ligands; at least 10²⁰ ligands; at least 10³⁰ ligands at least 10⁴⁰ ligands at least 10⁵⁰ ligands.

Typically the method comprises a step of co-incubating the ligand and the mixed cell population to facilitate binding of the ligand to an extraneous phenotypic cell expressing a binding partner of the ligand. The ligand and mixed cell population may be incubated at a temperature ranging from about 3°C to about 40°C. In some instances the ligand and the mixed cell population may be incubated at about 4°C; about 20°C; about 37°C.

A suitable length of time for incubating the ligand and the mixed population of cells may range from about 1 minute to about 60 minutes; about 5 minutes to about 50 minutes; about 10 minutes to about 40 minutes. In some instances the ligand and mixed cell population may incubated for about 10 minutes; about 15 minutes; about 20 minutes; about 25 minutes; about 30 minutes; about 60 minutes.

After contacting the mixed cell population with the ligand the cells may be washed one or more times with a suitable buffer to wash away non-specifically bound ligand. Suitable buffers include PBS, any commercially available isotonic buffer or media.

Separation of the ligand bound cells may be achieved by any method known in the art for separating mixtures. For example the ligand may be tagged with a detectable substance as described infra and separation may be achieved by cell sorting using a Fluorescent Activated Cell Sorter (FACS). As another example the ligand may be linked to a solid support as described, infra. The ligand bound cells may be precipitated by gravity. Alternatively an external force may be applied to the ligand bound cells such that the ligand bound cells separate from the other cells comprising the mixed population of cells. For example, the ligand may be linked to a magnetized solid support, such as a magnetic bead and the mixed cell population including the ligand bound cells may be exposed to a magnetic field such that the ligand bound cells separate from the other cells comprising the mixed population of cells.

In other instances a cell may be contacted with one or more ligands that bind specifically to a molecule expressed by a cell considered to be of an extraneous phenotype. In some instances the ligand may be conjugated with a chemical agent before it is contacted with the cell. In other instances the ligand may be bound to the cell first and then contacted with a chemical agent. Thus, the ligand bound cell may be targeted with a chemical agent that binds to the ligand bound cell, e.g., an agent that binds specifically to the ligand. In some embodiments one or more intervening ligands may be used. Thus a first ligand may bind the cell. A second ligand may bind the first ligand etc. The chemical agent may bind to any of the ligands bound to the cell. The chemical agent may be a toxin and its binding to the ligand bound cell may kill the cell. Examples of suitable toxins include diphtheria toxin, tetanus toxin and the like. The chemical agent may be a small molecule, a protein, a peptide, a nucleic acid, a carbohydrate, a lipid. In one embodiment where the ligand is an immunoglobulin the chemical agent may be complement.

Where separation is performed, the extraneous phenotypic cell may be harvested by removal or elution from the ligand. Elution of the extraneous phenotypic cells may be achieved by altering one or more of the binding conditions such as the pH of the buffer containing the bound cells or the salt concentration of the buffer containing the bound cells. Additionally, where separation is performed the target phenotype cell, e.g., the cardiomyocyte lineage cell may be harvested.

### Ligands

Any suitable ligand that binds specifically to a marker expressed by a cell having an extraneous phenotype may be used. The ligand may be comprised of a protein or peptide fragment of a full length protein. Suitable proteins include proteins or peptides that bind specifically to a molecule expressed on the surface of the extraneous phenotypic cell; however in some embodiments the ligand may bind an intracellular target. The ligand may be also comprised of a nucleic acid, a sugar, a lipid, a glycoprotein, or a lectin or a combination of any of these or a combination of a protein or peptide and any of these. Thus any ligand comprising a specific binding pair may be used as long as it binds specifically to a molecule expressed by the cells comprising the extraneous phenotype and does not specifically bind a target phenotypic cell or binds specifically to a portion of the extraneous phenotypic cells. Suitable ligands may have little to no non-specific binding to target phenotype cells such as cardiomyocyte lineage cells. In some instances the ligand may comprise a tag or a label that will facilitate removal or segregation of the ligand bound cells. Thus the ligand may be conjugated with a tag or a label. In other instances the ligand does not comprise a tag or a label.

In some instances one or more ligands may be used. Thus a first ligand may bind directly to a marker expressed by the extraneous phenotypic cell and a second ligand may then bind to the first ligand. Additional ligands which bind the second or any subsequent ligand are also contemplated. The second (or subsequent) ligand may comprise a tag which facilitates separation of the cells bound to the first ligand according to any method known in the art, e.g. cell sorting by FACS; precipitation of a ligand bound to a solid support; magnetic separation of ligand bound to a magnetic substrate; column chromatography over a ligand linked to a solid support. Of course multiple molecules expressed by an extra-phenotypic cell may be bound each with a distinct ligand and each of those ligands in turn may be contacted with one or more additional ligands.

In some instances the ligand may be an antibody. In some instances the antibody may comprise a variable region which binds specifically to an epitope found on an extraneous phenotypic cell and at least a portion of an Fc region to facilitate binding to a solid support or a second ligand. The antibody may be linked to a solid support as described infra. In some embodiments the solid support may be bead. In specific instances the bead may be magnetized.

In certain instances a suitable ligand may be one or more antibodies to an epitope expressed on the surface of an MSC cell. The epitope may be comprised of proteins, carbohydrates, sugars, and/or lipids expressed on the surface of an MSC cell. The epitope may be found on a marker expressed by an MSC. Markers found on MSC include CD90, CD140b, CD10, CD73, CD105, CD44 and Stro-1. Thus the antibody epitope may include epitopes found on one or more of the markers found on a MSC. Suitable ligands include one or more antibodies that specifically bind to one or more MSC markers. Ligands to markers found on MSC may be used alone or in combination with one or more additional ligands. Additional ligands may include ligands that bind specifically to molecules expressed by non-MSC cells having an extraneous phenotype.

In some instances the ligand may be one or more antibodies that bind to CD90. CD90 specific antibodies have been described in the literature, e.g. Craig et al. (1993) J. Exp Medicine 177:1331. Examples of suitable antibodies that bind to CD90 include commercially available antibodies such as: 5E10 (Life Technologies, Carlsbad, CA) THY1.1 (R&D Systems, Minneapolis, MN), AF9 (ABCAM, Cambridge, MA) and F15-42-1 (Abnova, Walnut, CA).

Ligands that bind to one or more markers expressed by undifferentiated pPS cells are also contemplated. These ligands may be used in combination with other ligands that bind specifically to markers expressed by other extraneous phenotypic cells such as MSC or CD90+ cells. Examples of suitable markers that may be used in the methods for reducing the number of extraneous phenotypic cells in a mixed population may include ligands, such as antibodies to TRA-1-60; TRA-1-81; SSEA 3; SSEA4; (See, Thomson 1998, Science 282:1145) Cripto, gastrin-releasing peptide (GRP) receptor, and podocalyxin-like protein (see U.S. Patent Application No. 10/388,578); EpCAM (WO 10/151,782). Thus antibodies that bind to epitopes found on undifferentiated cells may be used in combination with ligands that bind to cells that express CD90. Antibodies that bind to epitopes found on undifferentiated cells may be used in combination with ligands that bind to MSC.

The ligand may be coupled or linked covalently or non-covalently with a detectable substance to facilitate identification and/or isolation of bound cells. A detectable substance may include any compound, which when attached to a ligand, permits recognition of the presence of this ligand. The compound can comprise, for example, a radioactive molecule, a fluorescent molecule, a hapten, a carrier, an enzyme, an intervening molecule such as biotin, or a dye. The detectable substance, for example, may be a chemiluminescent material, or a bioluminescent material. The ligand may also be linked with a toxin such as any molecule that induces cell death, cell lysis etc.

In certain instances the ligand may be coupled or linked to a solid support. The solid support may comprise a bead, a gel, a monolith or a membrane. The solid support may comprise any material which can be linked to a ligand of interest (e.g., polystyrene, sepharose, sephadex). Solid supports may comprise any synthetic organic polymer such as polyacrylic, vinyl polymers, acrylate, polymethacrylate, polyacrylamide, polyacylonitriles, and polyolefins. Solid supports may also comprise a carbohydrate polymer, e.g., agarose, cellulose, hyaluronic acid, chitin, acyl gellan, dextran, carboxymethylcellulose, carboxymethyl starch, carboxymethyl chitin, poly(lactide-co-ethylene glycol). Solid supports may comprise, for example, nitrocellulose, nylon, polyvinylidene fluoride (PVDF) or carboxylated polyvinylidene (U.S. Patent No. 6,037,124). The solid support may be coated with polyvinyl benzyl dimethyl hydroxyethyl ammonium chloride, polyvinyl benzyl benzoyl aminoethyl dimethyl ammonium chloride, polyvinyl benzyl tributyl ammonium chloride, copolymers of polyvinyl benzyl trihexyl ammonium chloride and polyvinyl benzyl tributyl ammonium chloride, copolymers of polyvinyl benzyl dimethyl ammonium chloride and polyvinyl aminoethyl dimethyl ammonium chloride (U.S. Patent No. 5,336,596). Solid supports may comprise inorganic oxides, such as silica, zirconia, e.g., carbon clad zirconia (U.S. Patent No. 5,182,016), titania, ceria, alumina, manganese, magnesia (i.e., magnesium oxide), calcium oxide, controlled pore glass (CPG). Solid supports may also comprise combinations of some of the above-mentioned supports including, but not limited to, dextran-acrylamide. A solid support may be prepared to minimize non-specific interactions e.g., by coating it with one or more blocking proteins such as albumin, casein and the like. In some instances the ligand may be linked to both a solid support and a detectable substance.

In some instances : the ligand may be linked to a solid support such as a bead by mixing a suitable concentration of ligand and beads under conditions that facilitate covalent binding of the ligand to the solid support. Suitable concentrations of ligand and beads range from about 0.1 mg of ligand/mL of beads to about 20 mg of ligand/mL of bead; from about 0.5 mg of ligand/mL of beads to about 10 mg of ligand/mL of bead; from about 1.0 mg of ligand/mL of beads to about 5 mg of ligand/mL of bead. In some instances about 1 mg of ligand/mL of bead may be used. In other instances about 2 mg of ligand/mL of bead may be used. In some instances about 3 mg of ligand/mL of bead may be used. In other embodiments about 4 mg of ligand/mL of bead may be used. In still other embodiments about 5 mg of ligand/mL of bead may be used.

In some instances an antibody may be linked directly to the bead using known conjugation chemistry such as the formation of EHS ester. In other instances a specific binding partner of the antibody may be linked directly to the bead and the antibody in turn may bind to the specific binding partner thus linking it to the bead. The specific binding partner may bind to a region of the antibody that does not bind its specific epitope, e.g., a non variable region of the antibody. A suitable region may include the Fc region of the antibody. Examples of binding partners that bind to the Fc region of an antibody include protein A and protein G as well as immunoglobulins having variable regions that recognize the Fc region of the antibody bound to a marker expressed by an extraneous phenotype cell. Alternatively, the antibody may be conjugated to another molecule such as biotin and then coupled to a bead conjugated with streptavidin.

In one specific instance the ligand is an antibody to CD90 linked, e.g., covalently to a bead such as a magnetic bead. The antibody may be directly linked to the bead or indirectly linked by an intervening molecule such as protein A, protein G, biotin, streptavidin as described in the previous paragraph. The linker may be another antibody that specifically binds to a region of the antibody that does not bind to CD90, e.g., an Fc region. Alternatively, a short linker may serve to anchor the antibody to the bead thereby enhancing binding accessibility of the antibody. The linker may be comprised of one or more amino acids for example.

In another specific instance the ligand may be an antibody to TRA-1-60 linked covalently, e.g., to a bead such as a magnetic bead. The antibody may be directly linked to the bead or indirectly linked by an intervening molecule such as protein A, protein G, biotin, streptavidin as described in the previous paragraph. The linker may be another antibody that specifically binds to a region of the TRA-1-60 antibody that does not bind to TRA-1-60, e.g., an Fc region. Alternatively a short linker may serve to anchor the antibody to the bead thereby enhancing binding accessibility of the antibody. The linker may be comprised of one or more amino acids for example. The antibody to TRA-1-60 may have an IgG isotype in some embodiments. In other instances : the antibody to TRA-1-60 may have an IgM isotype.

In some instances a combination of one or more antibodies to markers expressed by MSC may be used to remove extraneous phenotypic cells from a mixed population of cells. In some instances the one or more antibodies to markers expressed by MSC may be combined with one or more antibodies to markers expressed by undifferentiated cells.

### Cell Populations

In certain instances the disclosure provides a mixed population of cells that is enriched for a targeted phenotype. Targeted phenotypes may include for example cardiac lineage cells; hematopoietic lineage cells. The targeted phenotype cells may be the in vitro differentiated progeny of a portion of a line of pPS cells. The mixed population of cells may be enriched for a targeted cell type by eliminating at least one cell having an extraneous phenotype from the mixed population of cells.

### 1. Extraneous Phenotypes

In some instances the extraneous phenotype may be the in vitro progeny of a line of pPS cells. In some instances the extraneous phenotypic cell is the differentiated in vitro progeny of a line of pPS cells. In some instances the extraneous phenotypic cell is the undifferentiated progeny of a line of pPs cells. In some embodiments the extraneous phenotypic cells may include both the in vitro differentiated progeny of a line of pPS cells and the in vitro undifferentiated progeny of a line of pPS cells.

In some instances extraneous phenotypic cells may include for example a cell having the morphology of an MSC, a cell expressing at least one marker expressed in or on or by a MSC cell, a cell capable of forming CFUs. Colonies of cells having a fibroblast like morphology are seen when MSCs are seeded in a tissue culture flask, typically at a low seeding density. In one instance the extraneous phenotype cell expresses CD90. In another instance the extraneous phenotype cell expresses CD73. In other instances the extraneous phenotype cell expresses CD105. In other instances the extraneous phenotype cell expresses CD 140b. In other instances the extraneous phenotype cell expresses CD10. In still other instances the extraneous phenotype cell expresses one or more markers chosen from CD90, CD10, CD140b, CD73, CD105, CD44, and Stro-1.

In other instances the extraneous phenotype includes cells expressing one or more markers expressed by, on, in or by a pPS cell. Examples of markers expressed in, on, or by a pPS cell include TRA-1-60, TRA-1-81; SSEA 3; SSEA 4; Oct 4. In some instances extraneous phenotype cells may include one or more of a MSC, a CD90+ cell, a CD73+ cell, a CD105+ cell, a CD10+ cell, a CD140b+ cell, a cell expressing one or more markers expressed by a pPS cell.

### 2.Targeted Phenotypes

Targeted phenotypic cells may be the in vitro differentiated progeny of pPS cells such as cardiomyocyte lineage cells. In some instances the target cell may be a mature target cell such as a mature cardiomyocyte. A mature cardiomyocyte will have contractile capability and express all of the markers found on a cardiomyocyte in vivo. In other embodiments the targeted cell type may a precursor cell such as a cardiomyocyte precursor. Precursor cells may express at least one marker expressed by, on or in a corresponding mature cardiomyocyte and/or possess one functional property of a mature cardiomyocyte; and/or have one or more morphological features found on a mature cardiomyocyte and/or have the ability to differentiate into a mature cardiomyocyte either in vivo or in vitro.

In certain instances markers expressed on, in or by targeted phenotype cells include one or more of the following: CD106, cardiac troponin I (cTnI), a subunit of troponin complex that provides a calcium sensitive molecular switch for the regulation of striated muscle contraction; cardiac troponin T (cTnT); Nkx2.5, a cardiac transcription factor expressed in cardiac mesoderm during early embryonic development, which persists in the developing heart; atrial natriuretic factor (ANF), a hormone expressed in developing heart and fetal cardiomyocytes but down-regulated in adults. Other suitable markers include myosin heavy chain (MHC), particularly the β chain which is cardiac specific; titin, tropomyosin, α sarcomeric actinin, and desmin; GATA-4, a transcription factor that is highly expressed in cardiac mesoderm and persists in the developing heart. Yet other suitable markers may include MEF 2A, MEF 2B, MEF 2C, MEF 2D; N-cadherin, which mediates adhesion among cardiac cells; connexin 43, which forms the gap junction between cardiomyocytes; β1 adrenoceptor (β1 AR); creatine kinase MB (CK MB) and myoglobin, which are elevated in serum following myocardial infarction; α cardiac actin.

Mixed populations of cells comprising cardiomyocyte lineage cells
may be obtained from any source. For example the mixed population of cells comprising cardiomyocyte lineage cells that are the in vitro progeny of a portion of a line of pPS cells may be obtained from a commercial vendor. Alternatively, a portion of a line of pPS cells may be differentiated in vitro to obtain a mixed population of cells comprising cardiomyocyte lineage cells according to any known method. Any cell expressing TRA-1-60, TRA-1-81, SSEA4 and SSEA3 may be used to obtain a population of cells comprising cardiomyocyte lineage cells according to the invention. Thus a population of cells comprising cardiomyocyte lineage cells may be obtained by differentiating a portion of a line of cells expressing TRA-1-60, TRA-1-81, SSEA4 and SSEA3 according to any of the methods described infra. Similarly enriched populations of cardiomyocyte lineage cells may be obtained by practicing any of the methods relating to enrichment of cell populations described infra on a population of cells comprising cardiomyocyte lineage cells wherein the cardiomyocyte lineage cells are the in vitro differentiated progeny of a line of cells expressing TRA-1-60, TRA-1-81, SSEA4 and SSEA3.

Suitable methods for obtaining cardiomyocyte lineage include the spontaneous or random differentiation of pPS cells into cardiomyocyte lineage cells, as well as the directed differentiation of pPS cells to cardiomyocyte lineage cells. Directed differentiation of pPS cells into cardiomyocyte lineage cells may comprise contacting the pPS cells (and/or the differentiated progeny of pPS cells) with one or more mitogens, growth factors, or cytokines. The one or more mitogens, growth factors, or cytokines can be added as a cocktail or added individually, or sequentially or any combination thereof. The one or more mitogens, growth factors, or cytokines may be removed from culture during the course differentiating the cells to cardiomyocyte lineage cells. The one or more mitogens, growth factors, or cytokines can be added back after the one or more mitogens, growth factors, or cytokines have been removed. Suitable mitogens, growth factors, or cytokines include activin and BMP.

In some instances a population of cells comprising cardiomyocyte lineage cells may be obtained by forming an embryoid body from a portion of a line of pPS cells. The method may further comprise contacting the cells with one or more mitogens, growth factors or cytokines. In some instances the embryoid body may be plated onto a solid support such as a tissue culture vessel. The vessel may be coated with a matrix such as gelatin. In other embodiments a population of cells comprising cardiomyocyte lineage cells may be obtained without forming an embryoid body from a portion of a line of pPS cells.

Methods of differentiating pPS in vitro into cardiomyocytes have been described, see, e.g., U.S. Patent Nos. 7,452,718; 7,425,448; U.S. Patent Publication Nos. 2005/0054092; 2007/0010012. As an example pPS cells may be contacted with activin followed by bone morphogenic protein (BMP) to differentiate pPS cells into cardiomyocytes. The pPS cells may be contacted by activin, followed by BMP in the absence of activin to obtain a population of cells comprising cardiomyocyte lineage cells. Thus in some instances a population of cells comprising cardiac lineage cells may be obtained by contacting a portion of a line of pPS cells with 100 ng/ml of activin A for 24 hours, removing the activin A from culture and then contacting the cells with 10 ng/ml of BMP, e.g., BMP4 for 4 days. The culture is then grown in media without adding any exogenous growth factors, mitogens or cytokines for 2-3 weeks. The media may be changed about every 2-3 days during the 2-3 week incubation.

### Enriched Cell Populations

As discussed above the disclosure provides cell populations enriched for a target phenotype such as a cardiomyocyte lineage cell. The cell populations may be enriched by reducing the number of extraneous phenotypic cells from the mixed population of cells. The extraneous phenotype cell may be a MSC. The extraneous phenotype cell may express CD90. The extraneous phenotype cell may expressCD73. The extraneous phenotype cell may express CD105. The extraneous phenotypic cells may be undifferentiated pPS cells. The extraneous phenotypic cells may be cells that express one or more markers expressed by a pPS cell, e.g., TRA-1-60, TRA-1-81, Oct4, SSEA3, SSEA4. The extraneous phenotypic cells may comprise a combination of one or more of the extraneous phenotypic cells described in this paragraph.

In certain instances the disclosure provides enriched populations of cell comprising a cardiomyocyte lineage cells wherein at least 1%, at least 5%, at least 10%, at least 15% at least 20% at least 25% at least 30%, at least 35%, at least 40%, at least 45%, at least 50%, at least 55%, at least 60%, at least 65%, at least 70%, at least 75%, at least 80%, at least 85%, at least 90%, at least 95%, at least 99% of the extraneous phenotypic cells have been removed from the cell population comprising the target cell phenotype.

In other instances the disclosure provides a mixed cell population comprising cardiomyocyte lineage cells, wherein at least one cell having an extraneous phenotype has been removed from the mixed population, and wherein the cardiomyocyte lineage cells comprise at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 99%, of the cells in the population after the at least one cell having an extraneous phenotype has been removed.

### Kits

In certain instances the disclosure provides a kit for depleting cells having an extraneous phenotype from a mixed population of cells, wherein the mixed population of cells comprises a targeted phenotypic cell such as cardiomyocyte lineage cells and extraneous phenotypic cells. Both cell types may be the in vitro progeny of a line of pPS cells. For example the targeted phenotypic cells may be comprised of the in vitro differentiated progeny of the pPS cells while the extraneous phenotypic cells may comprise differentiated progeny of the pPS cells, undifferentiated progeny of the pPS cells or a combination of both. The kit may comprise one or more ligands that specifically bind to one or more markers expressed by, on or in the cells comprising the extraneous phenotype. The one or more ligands may be provided in one or more containers. The ligands may be provided in a solution such as an aqueous buffer, e.g., an isotonic buffer, PBS or the like. Alternatively the one or more ligands may be provided in lyophilized form. The kit may include instructions for reconstituting the lyophilized ligand. The kit may include instructions for contacting the mixed population of cells with the ligand. The kit may include instructions for removing an extraneous phenotypic cell from a mixed population of cells comprising cardiomyocyte lineage cells wherein the cardiomyocyte lineage cells are the in vitro progeny of a line of pPS cells. The kit may include instructions for removing CD90+ cells from a population of cells comprising cardiomyocyte lineage cells that are the in vitro differentiated progeny of a line of pPS cells. The kit may include instructions for removing CD73+ cells from a population of cells comprising cardiomyocyte lineage cells that are the in vitro differentiated progeny of a line of pPS cells. The kit may include instructions for removing CD105+ cells from a population of cells comprising cardiomyocyte lineage cells that are the in vitro differentiated progeny of a line of pPS cells. The kit may include instructions for removing CD10+ cells from a population of cells comprising cardiomyocyte lineage cells that are the in vitro differentiated progeny of a line of pPS cells. The kit may include instructions for removing CD140b+ cells from a population of cells comprising cardiomyocyte lineage cells that are the in vitro differentiated progeny of a line of pPS cells.

The instructions may include a suitable concentration of ligand to use to deplete the extraneous phenotype. Optionally the kit may comprise one or more controls, e.g., a positive control cell type that will bind to the one or more ligands provided and a negative control cell type that will not bind to the one or more provided ligands. The kit may comprise a mixed population of cells comprising cardiomyocyte lineage cells and extraneous phenotypic cells such as CD90+ cells, MSCs or the like, wherein the percentage of extraneous phenotypic cells is already known, thus providing a means for comparing depletion efficiency in an unknown sample.

In some instances the ligand provided in the kit may be an antibody. The ligand may be an antibody that binds to a marker expressed by, on or in a cell comprising the extraneous phenotype. Examples of suitable antibodies include a CD90 antibody, a CD73 antibody, a CD105 antibody, a CD10 antibody, a CD140b antibody, a CD44 antibody, a Stro-1 antibody, a TRA-1-60 antibody, an antibody to TRA-1-81, an antibody to SSEA3, and an antibody to SSEA4. The antibody may be any isotype e.g., IgG, IgM, IgA, IgE, IgD. In one embodiment the antibody may be an IgG. In one instance the antibody may be an IgM that binds to TRA-1-60. In one instance the antibody may be an IgG that binds to TRA-1-60. In one instance a combination of different antibodies may be used, e.g., a plurality of the types of antibodies described in this paragraph. Thus a plurality of antibodies directed to a single antigen (but different epitopes) may be used. A plurality of antibodies directed to a plurality of antigens may be used.

The kit may comprise a solid support for the ligand. Solid supports are described in detail infra. The ligand may be provided already linked to the solid support in a single container. Alternatively the kit may provide the antibody and solid support unlinked to one another. The solid support and the ligand may thus be provided in separate containers. The kit may provide instructions and one or more reagents for linking the ligand to the solid support.

The kit may further comprise one or more detectable substances which may be linked to the one or more ligands provided in the kit. Detectable substances are described in detail infra. The one or more detectable substances may each be provided in one or more separate containers along with instructions for linking the one or more detectable substances to the one or more ligands.

The kit may provide a means for applying an external force to the mixed population of cells after it has been contacted with the ligand bound to a solid support such that separation of the ligand bound extraneous phenotypic cells from the mixed population of cells is facilitated. The external force may be a magnetic field.

### Uses of Enriched Cell Populations

This disclosure provides a method to produce large numbers of cells enriched for a target phenotype, e.g. cardiomyocyte lineage cells. These cell populations can be used for a number of important therapeutic, research, development, and commercial purposes. Because the populations provided are enriched for a target phenotype they will be more suitable for all of the uses described infra when compared to cell populations that have not been so enriched.

### Screening

The enriched target cell populations of this disclosure can be used commercially to screen for factors (such as solvents, small molecule drugs, peptides, oligonucleotides) or environmental conditions (such as culture conditions) that affect the characteristics of such cells and their various progeny. Characteristics may include phenotypic or functional traits of the cells. Other characteristics that may be observed include the differentiation status of the cells; the maturity of the cells and the survival rate of the cells after exposure to the factor. Thus the enriched population of cells comprising cardiomyocyte lineage cells may be contacted with one or more factors and the effects of the factors may be compared to an aliquot of the same cells that has not been contacted with the factors.

Other screening applications of this disclosure relate to the testing of pharmaceutical compounds for their effect on enriched target cell populations. Screening may be done either because the compound is designed to have a pharmacological effect on the cells, or because a compound is designed to have effects elsewhere and may have unintended side effects on cells of the target phenotype. Other screening applications could include screening compounds for carcinogenic or other toxic effects. The screening can be conducted using any of the precursor cells or terminally differentiated/mature cells of the invention in order to determine if the target compound has a beneficial or harmful effect on the target cell. Using the enriched target populations described infra will provide for more accurate screening results.

The reader is referred generally to the standard textbook In vitro Methods in Pharmaceutical Research, Academic Press, 1997. Assessment of the activity of candidate pharmaceutical compounds generally involves combining the enriched target cells of this invention with the candidate compound, either alone or in combination with other drugs. The investigator determines any change in the morphology, marker phenotype as described infra, or functional activity of the cells, that is attributable to the compound (compared with untreated cells or cells treated with an inert compound), and then correlates the effect of the compound with the observed change.

Cytotoxicity can be determined in the first instance by the effect on cell viability, survival, morphology, and the expression of certain markers and receptors. Effects of a drug on chromosomal DNA can be determined by measuring DNA synthesis or repair. [³H]-thymidine or BrdU incorporation, especially at unscheduled times in the cell cycle, or above the level required for cell replication, is consistent with a drug effect. Unwanted effects can also include unusual rates of sister chromatid exchange, determined by metaphase spread. The reader is referred to A. Vickers (pp 375-410 in In vitro Methods in Pharmaceutical Research, Academic Press, 1997) for further elaboration.

Where an effect is observed, the concentration of the compound can be titrated to determine the median effective dose (ED₅₀).

### Gene Expression

The cells of this disclosure can be used to prepare a cDNA library relatively uncontaminated with cDNA preferentially expressed in cells from other lineages. For example, cardiomyocyte lineage cells according to this invention are collected by centrifugation at 1000 rpm for 5 min, and then mRNA is prepared from the pellet by standard techniques (Sambrook et al., supra). After reverse transcribing into cDNA, the preparation can be subtracted with cDNA from undifferentiated pPS cells, other progenitor cells, or end-stage cells from the cardiomyocyte or any other developmental pathway.

Expression patterns of the enriched target cells may be compared with other cell types by microarray analysis, reviewed generally by Fritz et al. Science 288:316, 2000. Such libraries would be especially well suited for studying gene expression in target cells compared to the undifferentiated pPS cells from which they were derived. Because these cells share essentially identical genomes comparisons in gene expression using for example subtractive hybridization can be made with little or no background noise. Reducing the number of extraneous phenotypic cells within a cell population will provide improved signal to noise ratios in comparing gene expression in two populations of cells, e.g., differentiated target progeny and the parent pPS cell line giving rise to the differentiated target cells.

The use of microarray in analyzing gene expression is reviewed generally by Fritz et al Science 288:316, 2000; Microarray Biochip Technology, L Shi,. An exemplary method is conducted using a Genetic Microsystems array generator, and an Axon GenePix™ Scanner. Microarrays are prepared by first amplifying cDNA fragments encoding marker sequences to be analyzed, and spotted directly onto glass slides. To compare mRNA preparations from two cells of interest, one preparation is converted into Cy3-labeled cDNA, while the other is converted into Cy5-labeled cDNA. The two cDNA preparations are hybridized simultaneously to the microarray slide, and then washed to eliminate non-specific binding. The slide is then scanned at wavelengths appropriate for each of the labels, the resulting fluorescence is quantified, and the results are formatted to give an indication of the relative abundance of mRNA for each marker on the array.

### Animal testing

This disclosure also provides for the use of enriched target cells of the invention to enhance tissue maintenance or repair of tissue function for any perceived need, such as an inborn error in metabolic function, the effect of a disease condition, or the result of significant trauma.

To determine the suitability of cell compositions for therapeutic administration, the enriched target cells can first be tested in a suitable subject such as a rat, mouse, guinea pig, rabbit, cow, horse, sheep, pig, dog, primate or other mammal. At one level, cells are assessed for their ability to survive and maintain their phenotype in vivo. Cell compositions may be administered to immunodeficient animals (such as nude mice, or animals rendered immunodeficient chemically or by irradiation). Tissues are harvested after a period of regrowth, and assessed as to whether pluripotent stem derived cells are still present. Functional tests as are known in the art may be performed.

Cell survival may be monitored by administering cells that express a detectable label (such as green fluorescent protein, or β-galactosidase); that have been prelabeled (for example, with BrdU or [³H]thymidine), or by subsequent detection of a constitutive cell marker (for example, using human-specific antibody). The presence and phenotype of the administered cells can be assessed by immunohistochemistry or ELISA using human-specific antibody, or by RT-PCR analysis using primers and hybridization conditions that cause amplification to be specific for human polynucleotides, according to published sequence data.

In certain instances the enriched target cells of the disclosure may be tested functionally using a known animal model for a particular disease. Where the target cell type is a cardiomyocyte lineage cell, for example, one of several models for heart disease and or infarction can be used. Suitable animal models include, but are not limited to pigs, guinea pigs, rats and mice. Hearts can be cryoinjured by placing a precooled aluminum rod in contact with the surface of the anterior left ventricle wall (Murry et al., J. Clin. Invest. 98:2209, 1996; Reinecke et al., Circulation 100:193, 1999; U.S. Patent No. 6,099,832; Reinecke et al., Circ Res., Epub Mar 2004). In larger animals, cryoinjury can be effected by placing a 30-50 mm copper disk probe cooled in liquid N₂ on the anterior wall of the left ventricle for ∼20 min (Chiu et al., Ann. Thorac. Surg. 60:12, 1995). Infarction can be induced by ligating the left main coronary artery (Li et al., J. Clin. Invest. 100:1991, 1997) or by using an ameroid constriction device that gradually swells to occlude an artery. Injured sites are treated with cell preparations of this invention, and the heart tissue is examined by histology for the presence of the cells in the damaged area. Cardiac function can be monitored by determining such parameters as left ventricular end-diastolic pressure, developed pressure, rate of pressure rise, and rate of pressure decay.

### Therapeutic use in humans

After adequate testing, enriched target cells of this disclosure can be used for tissue reconstitution or regeneration in a human patient or other subject in need of cell therapy. The cells are administered in a manner that permits them to graft or migrate to the intended tissue site and reconstitute or regenerate the functionally deficient area. Thus enriched target cell populations comprising cardiomyocyte lineage cells may be administered to the heart.

Administration of the population of cells may be achieved by any method known in the art. For example the cells may be administered surgically directly to the organ or tissue in need of a cellular transplant. Alternatively non-invasive procedures may be used to administer the cells to the subject. Examples of non-invasive delivery methods include the use of syringes and/or catheters to deliver the cells into the organ or tissue in need of cellular therapy.

The patient receiving an allograft of enriched target cells of the invention may be treated to reduce immune rejection of the transplanted cells. Methods contemplated include the administration of traditional immunosuppressive drugs like tacrolimus, cyclosporin A (Dunn et al., Drugs 61:1957, 2001), or inducing immunotolerance using a matched population of pluripotent stem derived cells (WO 02/44343; U.S. Patent No. 6,280,718; WO 03/050251). Alternatively a combination of anti-inflammatory (such as prednisone) and immunosuppressive drugs may be used.

The enriched target cells of this disclosure can be supplied in the form of a pharmaceutical composition, comprising an isotonic excipient prepared under sufficiently sterile conditions for human administration. To reduce the risk of cell death upon engraftment, the cells may be treated by heat shock or cultured with -0.5 U/mL erythropoietin ∼24 hours before administration.

For general principles in medicinal formulation, the reader is referred to Allogeneic Stem Cell Transplantation, Lazarus and Laughlin Eds. Springer Science+Business Media LLC 2010; and Hematopoietic Stem Cell Therapy, E.D. Ball, J. Lister & P. Law, Churchill Livingstone, 2000. Choice of the cellular excipient and any accompanying elements of the composition will be adapted in accordance with the route and device used for administration. The composition may also comprise or be accompanied with one or more other ingredients that facilitate the engraftment or functional mobilization of the enriched target cells. Suitable ingredients may include matrix proteins that support or promote adhesion of the target cell type or that promote vascularization of the implanted tissue.

This disclosure also includes a reagent system, comprising a set or combination of cells that exist at any time during manufacture, distribution, or use. The cell sets comprise any combination of two or more cell populations described in this disclosure, exemplified but not limited to a type of differentiated pluripotent stem-derived cell such as a population of cardiomyocyte lineage cells, in combination with undifferentiated primate pluripotent stem cells or other differentiated cell types, often sharing the same genome. The genome of the differentiated progeny and the parental pPS cell line may be at least 95% identical, at least 96% identical, at least 97% identical; at least 98% identical, at least 99% identical, at least 99.5% identical; at least 99.6% identical; at least 99.7% identical; at least 99.8% identical; at least 99.9% identical. The genome of the differentiated progeny and the parental pPS cell line may be identical. Each cell type in the set may be packaged together, or in separate containers in the same facility, or at different locations, at the same or different times, under control of the same entity or different entities sharing a business relationship.

Pharmaceutical compositions of this disclosure may optionally be packaged in a suitable container with written instructions for a desired purpose, such as the reconstitution of cardiomyocyte-lineage cell function.

### Antibodies

The differentiated cells of this disclosure can also be used to prepare antibodies that are specific for markers of the enriched target cells. Polyclonal antibodies can be prepared by injecting a vertebrate animal with cells of this disclosure in an immunogenic form. Production of monoclonal antibodies is described in such standard references as Harlow & Lane (1988) Antibodies: A Laboratory Manual, U.S. Patent Nos. 4,491,632, 4,472,500 and 4,444,887, and Methods in Enzymology 73B:3 (1981).

### Primate Pluripotent Stem cells

The present disclosure provides methods for enriching target cells differentiated in vitro from pPS cells. pPS cells include any primate pluripotent cell. A pluripotent cell will, under appropriate growth conditions, be able to form at least one cell type from each of the three primary germ layers: mesoderm, endoderm and ectoderm. Typically, the pPS cells are not derived from a malignant source. pPS cells will form teratomas when implanted in an immuno-deficient mouse, e.g., a SCID mouse. For reference, the pPS cells may be obtained from an established cell line. Established reference cell lines are available from public cell banks such as WiCell and the UK Stem Cell Bank.

Under the microscope, pPS cells appear with high nuclear/cytoplasmic ratios, prominent nucleoli, and compact colony formation with poorly discernable cell junctions. pPS cells typically express the stage-specific embryonic antigens (SSEA) 3 and 4, and markers detectable using antibodies designated TRA-1-60 and TRA-1-81. Undifferentiated reference human embryonic stem cells also typically express the transcription factor Oct-3/4, Cripto, gastrin-releasing peptide (GRP) receptor, podocalyxin-like protein (PODXL), nanog and telomerase reverse transcriptase, e.g., hTERT (US 2003/0224411 A1), as detected by RT-PCR.

pPS cells that may be used in any of the instances of the disclosure include, but are not limited to, reference embryonic stem cells such as human reference embryonic stem cells (hES). Reference embryonic stem cells may be chosen from reference embryonic stem cell lines. A large number of reference embryonic stem cell lines have been established including, but not limited to, H1, H7, H9, H13 or H14 (Thompson, (1998) Science 282:1145); hESBGN-01, hESBGN-02, hESBGN-03 (BresaGen, Inc., Athens, GA); HES-1, HES-2, HES-3, HES-4, HES-5, HES-6 (from ES Cell International, Inc., Singapore); HSF-1, HSF-6 (from University of California at San Francisco); I 3, I 3.2, 13.3, I 4, I 6, I 6.2, J 3, J 3.2 (derived at the Technion-Israel Institute of Technology, Haifa, Israel); UCSF-1 and UCSF-2 (Genbacev et al., (2005) Fertil. Steril. 83(5):1517); lines HUES 1-17 (Cowan et al., (2004) NEJM 350(13):1353); and line ACT-14 (Klimanskaya et al., (2005) Lancet, 365(9471):1636).

pPS cells suitable for use in any of the instances of the disclosure also include induced primate pluripotent stem (iPS) cells. iPS cells refer to cells that are genetically modified, e.g., by transfection with one or more appropriate vectors, such that they attain the phenotype of a pPS cell (Takahashi et al. (2007) Cell 131(5):861; Yu et al. (2007) Science 318:1917). Alternatively, iPS cells may be obtained by reprogramming adult cells by contacting them with a protein cocktail that induces the cells to reprogram such that they have phenotypic and morphological traits associated with blastocyst derived pluripotent stem cells see, Kim et al. (2009) Cell Stem Cell 4(6):472. Additionally iPS cells may be obtained by treating somatic cells with a cocktail of small molecules (Ichida et al. (2009) Cell Stem Cell 5:491). Other methods of obtaining iPS cells include treating somatic cells with microRNAs (Judson et al. (2009) Nature Biotech 27:459). Phenotypic traits attained by these reprogrammed cells include morphology resembling pluripotent stem cells isolated from a blastocyst, as well as expression of surface antigens, gene expression and telomerase activity found in pPS cells. The iPS cells may have the ability to differentiate into at least one cell type from each of the primary germ layers: ectoderm, endoderm and mesoderm. The iPS cells may also form teratomas when injected into immunodeficient mice, e.g., SCID mice. (Takahashi et al., (2007) Cell 131(5):861; Yu et al., (2007) Science 318:1917).

pPS cells include any cell expressing the markers SSEA3; SSEA4; TRA-1-60 and TRA-181.

### Culture Conditions for Primate Pluripotent Stem Cells

In certain instances, pPS cells used in the present disclosure may have been derived in a feeder-free manner (see, *e*.*g*., Klimanskaya et al., (2005) Lancet 365(9471):1636). In certain embodiments the pPS may be cultured prior to use in a serum free environment.

pPS cells may be cultured using a variety of substrates, media, and other supplements and factors known in the art. In some instances a suitable substrate may be comprised of a matrix including one or more of the following: laminin, collagen, fibronectin, vitronectin, heparin sulfate proteoglycan and/or peptide fragments derived from any of the foregoing proteins. In some instances the matrix may comprise a soluble extract of the basement membrane from a murine EHS sarcoma which is commercially available as Matrigel™ (BD Biosciences, San Jose, CA). In other instances the matrix may comprise one more isolated matrix proteins of human, humanized, or murine origin, e.g., CELLstart™ (Invitrogen, Carlsbad, CA). Matrix proteins may include any protein found in an in vivo extra-cellular matrix. In still other instances a suitable substrate may be comprised of one or more polymers such as one or more acrylates. The polymers may include one or more proteins or peptide fragments derived from a protein found in vivo in the extra-cellular matrix. In one particular embodiment the substrate is comprised of one or more acrylates and a conjugated vitronectin peptide (see, e.g. U.S Patent Publication No. 2009/0191633; U.S Patent Publication No. 2009/0191626; U.S Patent Publication No. 2009/0203065). pPS cells can be propagated continuously in culture, using culture conditions that promote proliferation while inhibiting differentiation.

Exemplary medium may be made with 80% DMEM (such as Knock-Out DMEM, Gibco), 20% of either defined fetal bovine serum (FBS, Hyclone) or serum replacement (US 2002/0076747 A1, Life Technologies Inc.), 1% non-essential amino acids, 1 mM L-glutamine, and 0.1 mM β-mercaptoethanol. Other suitable media include serum free defined media such as X-VTVO™ 10 (Lonza, Walkersville, MD). Still other commercially available media formulations that may be used in certain embodiments of the invention include X-VIVO™ 15 (Lonza, Walkersville, MD); mTeSR™ (Stem Cell Technologies, Vancouver, CA); hTeSR™ (Stem Cell Technologies, Vancouver, CA), StemPro™ (Invitrogen, Carlsbad, CA) and Cellgro™ DC (Mediatech, Inc., Manassas, VA).

In certain instances, pPS cells may be maintained in an undifferentiated state without added feeder cells (see, *e*.*g*., (2004) Rosler et al., Dev. Dynam. 229:259). Feeder-free cultures are typically supported by a nutrient medium containing factors that promote proliferation of the cells without differentiation (see, e.g., U.S. Patent No. 6,800,480). In certain embodiments, conditioned media containing such factors may be used. Conditioned media may be obtained by culturing the media with cells secreting such factors. Suitable cells include irradiated (∼4,000 rad) primary mouse embryonic fibroblasts, telomerized mouse fibroblasts, or fibroblast-like cells derived from pPS cells (U.S. Patent No. 6,642,048). Medium can be conditioned by plating the feeders in a serum free medium such as KO DMEM supplemented with 20% serum replacement and 4 ng/mL bFGF. Medium that has been conditioned for 1-2 days may be supplemented with further bFGF, and used to support pPS cell culture for 1-2 days (see. e.g., WO 01/51616; Xu et al., (2001) Nat. Biotechnol. 19:971).

Alternatively, fresh or non-conditioned medium can be used, which has been supplemented with added factors (like a fibroblast growth factor or forskolin) that promote proliferation of the cells in an undifferentiated form. Exemplary is a base medium like X-VIVO™ 10 (Lonza, Walkersville, MD) or QBSF™-60 (Quality Biological Inc. Gaithersburg, MD), supplemented with bFGF at 40-80 ng/mL, and optionally containing SCF (15 ng/mL), or Flt3 ligand (75 ng/mL) (see, *e.g*., Xu et al., (2005) Stem Cells 23(3):315). These media formulations have the advantage of supporting cell growth at 2-3 times the rate in other systems (see, *e*.*g*., WO 03/020920). In some instances pPS cells such as hES cells may be cultured in a media comprising bFGF and TGFβ. Suitable concentrations of bFGF include about 80 ng/ml. Suitable concentrations of TGFβ include about 0.5ng/ml.

In some instances, the primate pluripotent stem cells may be plated at >15,000 cells cm⁻² (optimally 90,000 cm⁻² to 170,000 cm⁻²). Typically, enzymatic digestion may be halted before cells become completely dispersed (e.g., about 5 minutes with collagenase IV). Clumps of about 10 to about 2,000 cells may then be plated directly onto a suitable substrate without further dispersal. Alternatively, the cells may be harvested without enzymes before the plate reaches confluence by incubating the cells for about 5 minutes in a solution of 0.5 mM EDTA in PBS or by simply detaching the desired cells from the plate mechanically, such as by scraping or isolation with a fine pipette or a cell scraper. After washing from the culture vessel, the cells may be plated into a new culture without further dispersal. In a further illustration, confluent human embryonic stem cells cultured in the absence of feeders may be removed from the plates by incubating with a solution of 0.05% (wt/vol) trypsin (Gibco®, Carlsbad, CA) and 0.05 mM EDTA for 5-15 minutes at 37°C. The remaining cells in the plate may be removed and the cells may be triturated into a suspension comprising single cells and small clusters, and then plated at densities of 50,000-200,000 cells cm⁻² to promote survival and limit differentiation.

In certain instances, pPS cells may be cultured on a layer of feeder cells, typically fibroblasts derived from embryonic or fetal tissue (Thomson et al. (1998) Science 282:1145). In certain instances, those feeder cells may be derived from human or murine source. Human feeder cells can be isolated from various human tissues or derived by differentiation of human embryonic stem cells into fibroblast cells (see, *e*.*g*., WO 01/51616) In differentiation of human reference feeder cells that may be used include, but are not limited to, placental fibroblasts (see, *e*.*g*., Genbacev et al. (2005) Fertil. Steril. 83(5):1517), fallopian tube epithelial cells (see, *e*.*g*., Richards et al. (2002) Nat. Biotechnol., 20:933), foreskin fibroblasts (see, *e*.*g*., Amit et al. (2003) Biol. Reprod. 68:2150), uterine endometrial cells (see, *e*.*g*., Lee et al. (2005) Biol. Reprod. 72(1):42).

In the practice of the present invention, there are various solid surfaces that may be used in the culturing of cells. Those solid surfaces include, but are not limited to, standard commercially available cell culture plates such as 6-well, 24-well, 96-well, or 144-well plates. Other solid surfaces include, but are not limited to, microcarriers and disks. In certain instances, the microcarriers may be used in stirred-tank bioreactors for attachment of the cells. In certain instances, the microcarriers are beads. Those beads come in various forms such as Cytodex Dextran microcarrier beads with positive charge groups to augment cell attachment, gelatin/collagen-coated beads for cell attachment, and macroporous microcarrier beads with different porosities for attachment of cells. The Cytodex dextran, gelatin-coated and the macroporous microcarrier beads are commercially available (Sigma-Aldrich, St. Louis, MO or Solohill Engineering Inc., Ann Arbor, MI). In certain instances, the beads are 90-200 µm in size with an area of 350-500 cm². Beads may be composed of a variety of materials such as, but not limited to, glass or plastic. Disks are sold by companies such as New Brunswick Scientific Co, Inc. (Edison, NJ). In certain embodiments, the disks are Fibra-cel Disks, which are polyester/polypropylene disks. A gram of these disks provide a surface area of 1200 cm².

The solid surface suitable for growing pPS cells may be made of a variety of substances including, but not limited to, glass or plastic such as polystyrene, polyvinylchloride, polycarbonate, polytetrafluorethylene, melinex, or thermanox. In certain instances,
the solid surfaces may be three-dimensional in shape. Exemplary three-dimensional solid surfaces are described, *e.g*., in US 2005/0031598.

In certain instances, the cells are in a single-cell suspension during the methods of the invention. The single-cell suspension may be performed in various ways including, but not limited to, culture in a spinner flask, in a shaker flask, or in a fermentors. Fermentors that may be used include, but are not limited to, Celligen Plus (New Brunswick Scientific Co, Inc., Edison, NJ), and the STR or the Stirred-Tank Reactor (Applikon Inc., Foster City, CA). In certain embodiments, the bioreactors may be continuously perfused with media or used in a fed-batch mode. Other suitable bioreactors include the Wave Bioreactor bags (GE Healthcare, Piscataway, NJ). Bioreactors come in different sizes including, but not limited to 2.2 liter, 5 liter, 7.5 liter, 14 liter or 20 liter, 100 liter, 100 liter, 10,000 liter or larger.

### General Techniques

For further elaboration of general techniques useful in the practice of this invention, the practitioner can refer to standard textbooks and reviews in cell biology, tissue culture, embryology, developmental biology, immunology, neurobiology, endocrinology, cardiology and the like.

With respect to tissue and cell culture and reference embryonic stem cells, the reader may wish to refer to any of numerous publications available in the art, e.g., Teratocarcinomas and Embryonic Stem cells: A Practical Approach (E.J. Robertson, Ed., IRL Press Ltd. 1987); Guide to Techniques in Mouse Development (P.M. Wasserman et al., Eds., Academic Press 1993); Embryonic Stem Cell Differentiation in Vitro (M.V. Wiles, Meth. Enzymol. 225:900, 1993*);* Properties and Uses of Embryonic Stem Cells: Prospects for Application to Human Biology and Gene Therapy (P.D. Rathjen et al., Reprod. Fertil. Dev. 10:31, 1998; and R.I. Freshney, Culture of Animal Cells, Wiley-Liss, New York, 2000).

Where derived from an established line of pPS cells, the cell populations and isolated cells of this invention can be characterized as having the same genome as the line from which they are derived. This means that the chromosomal DNA will be essentially identical by RFLP or by SNP analysis between the pPS cells and the differentiated progeny cells (assuming the cells have not been genetically manipulated by the human hand). It is contemplated that relatively minute changes in the genome may occur over time, e.g. in the non-coding regions, however overall the genetic identity will be substantially maintained between the parent cell line and any progeny, e.g. differentiated progeny. Typically the level of genetic identity will be similar to genetic identity observed between identical twins.

### Genetic alteration of differentiated cells

The cells of this invention can be made to contain one or more genetic alterations by genetic engineering of the cells either before or after differentiation (US 2002/0168766 A1). For example in some embodiments, the cells can be processed to increase their replication potential by genetically altering the cells to express telomerase reverse transcriptase, either before or after they progress to restricted developmental lineage cells or terminally differentiated cells (US 2003/0022367 A1).

The cells of this disclosure can also be genetically altered in order to enhance their ability to be involved in modulating a specific therapeutic function, or to deliver a therapeutic gene to a site of administration. A vector is designed using the known encoding sequence for the desired gene, operatively linked to a promoter that is either active in all cell types or specifically active in the differentiated cell type. Alternatively the promoter may be an inducible promoter that permits for the timed expression of the genetic alteration. For example the cells may be genetically engineered to express a cytokine that modulates a cardiac function.

In the following Examples all experiments utilizing reference human embryonic cells (hES) cells were performed using established publicly available reference hES cell line.

### EXAMPLES

### Reference Example 1: Mesenchymal stem cells are present in cardiomyocyte lineage cells differentiated in vitro from reference hES cells

Mesenchymal stem cells (MSCs) are multipotent cells that possess a unique capacity to differentiate into specific cell types. The identification and characterization of MSCs are generally characterized using three parameters: 1) Adherence to plastic under standard culture conditions; 2) Cell surface expression of CD73, CD90, and CD105; and 3) In vitro differentiation into osteoblasts, chondrocytes, or adipocytes. These parameters were used to evaluate for the presence of MSCs in a preparation of cardiomyocyte lineage cells which were obtained by the in vitro differentiation of reference human embryonic stem cells.

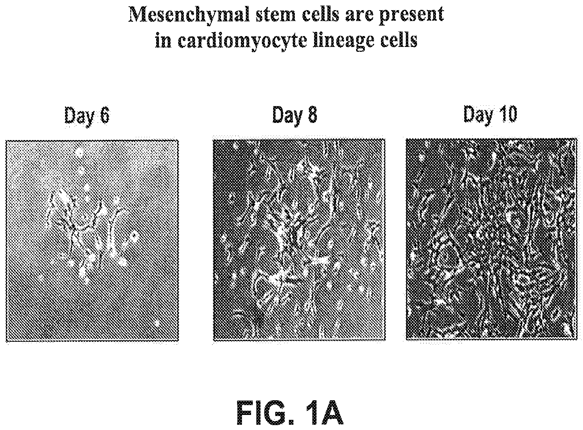

For the initial identification of MSCs in the cardiomyocyte lineage cell preparation, adherence culture methods were utilized. Cardiomyocyte lineage cells were added to 6-well standard tissue culture plates (Corning Life Sciences, Corning, NY) containing MSC maintenance medium (MSCGM™) supplemented with MSCGM™ SingleQuots® (Lonza, Walkersville, Maryland). Cells were cultured for 2hrs at 37°C 5% CO₂. After 2 hrs, the non-adherent cells were washed 1X with medium and fresh medium was added to the wells (4mL/well). The cells were cultured for an additional 14 days and medium was completely exchanged every 4-5 days. At different time points post culture, bright field images were captured (Figure 1A). The images are a representation of the proliferating cells in culture possessing fibroblast-type morphology. By day 10, tight colonies of proliferating cells were evident that are typical of MSC colonies.

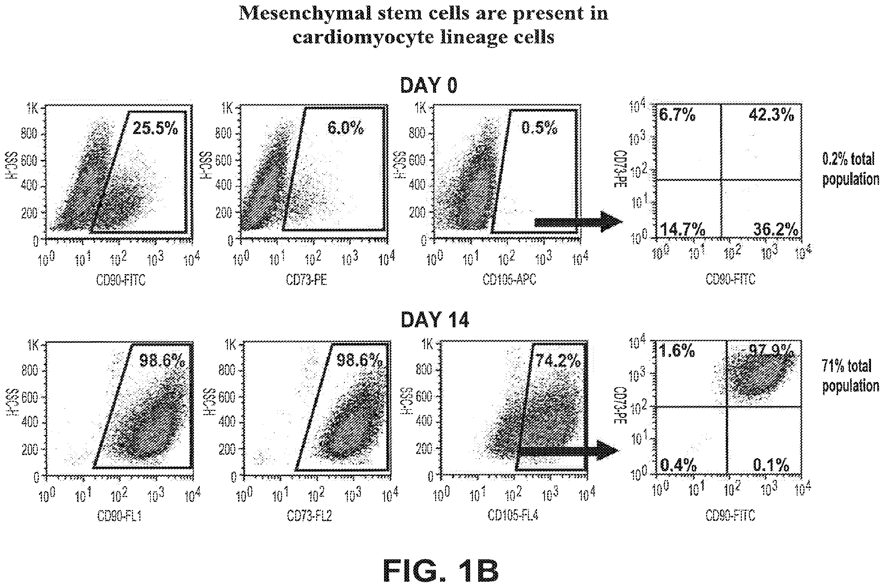

The cell's surface phenotype of the proliferating cell colonies in culture was evaluated for MSC associated marker expression. After 14 days in MSC medium culture, cells were harvested for cell surface phenotype analysis. The cells were stained for 30 minutes with antibodies against CD73 PE conjugated 10µl/test (BD Biosciences, San Jose, CA), CD90 FITC conjugated 0.5µg/test (BD Biosciences, San Jose, CA), and CD105 APC conjugated, 10µl/test (Biolegend, San Diego, CA. Cells were washed 2X in 1% BSA 1X PBS and analyzed using flow cytometric analysis. FACSCalibur™ (Becton Dickinson, Franklin Lakes, NJ). Single positives percentages of each marker, as well as the percentage of cells expressing all 3 markers were evaluated at day 0 and day 14 (Figure 1B). At day 0, a small portion of cells (0.2% of the total population) expressing all three markers, was observed. By day 14, 71% of the cells were expressing all 3 markers, suggesting the population of cells that expanded in culture likely represented MSCs.

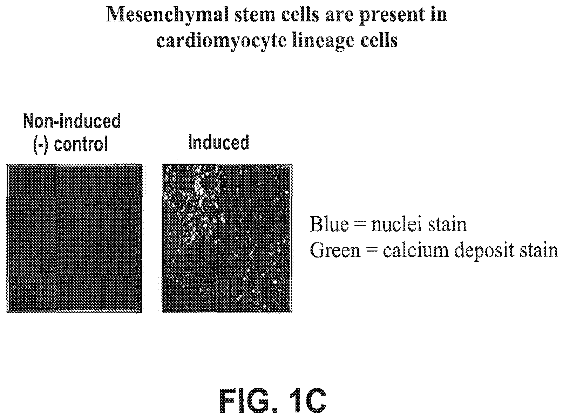

To further confirm that these cells represented MSCs, the cells were cultured in osteoblasts differentiation medium (Lonza, Walkersville, Maryland) for an additional 14 days to determine their differentiation potential. Medium was exchanged and completely replaced every 3 days. To test for the presence of differentiated MSCs into osteoblasts, the OsteoImage Kit (Lonza, Walkersville, Maryland) was used according to the manufacturer's instructions to detect the presence of calcium deposits in the form of mineralized nodules composted of inorganic hydroxyapatite and organic components that include collagen type I (Figure 1C). The presence of calcium deposits was detected from the differentiated cultures (green florescent stain), and not observed in the non-differentiated negative controls (MSCs not treated with induction media), suggesting cells present in the cardiomyocyte lineage cell preparation that expanded and proliferated *in vitro* were MSCs.

### Reference Example II. Depletion of CD90 positive cells using indirect isolation depletes MSCs from cardiomyocyte lineage cells differentiated in vitro from reference hES cells

CD90, a marker expressed by MSCs, was used as a target antigen for depletion of MSCs from cardiomyocyte lineage cells differentiated in vitro from reference hES cells. To deplete CD90+ cells from the cardiomyocyte lineage cells, the Miltenyi microbead system was utilized (Miltenyi Biotec, Auburn, CA). The cardiomyocyte lineage cells were resuspended in depletion buffer (PBS + 0.5% BSA + 2 mM EDTA) and mouse anti-human CD90 antibody conjugated to PE, (10µl/1e⁶ cells) (BD Biosciences, San Jose, CA) at a final cell concentration of 4e⁷ cells/mL. Cells were incubated with the antibody for 20 minutes at 4°C and then washed 1X and resuspended with depletion buffer at 1e⁷cells/80µL. Following the manufacturer's instructions, anti-PE micro beads (Miltenyi Biotec, Auburn, CA) were added to the cells at 20µL per 1e⁷ cells. Cells were incubated for 15 minutes at 4°C and then washed 1X and resuspended with depletion buffer using 500µL of buffer for cell numbers up to 1e⁸ cells. Cells were added to a MS depletion column (Miltenyi Biotec, Auburn, CA) that had previously been washed with 3mL of depletion buffer, and then attached to a MidiMACS Separator (Miltenyi Biotec, Auburn, CA). Unlabeled CD90 negative cells passed through the column and were collected in a sterile 15mL polypropylene tube (Corning Life Sciences, Corning, NY). The column was washed 3X washes with 3mL of depletion medium. Each successive wash was applied to the column once the column was empty. The MS depletion column was removed from the separator and 4mL of depletion buffer was added to the column. To isolate the CD90 positive cells bound to the column, a plunger was used to flush out the cells into a sterile 15mL polypropylene tube. Cells were washed 2X with depletion buffer, and resuspended in MSC maintenance medium (Lonza, Walkersville, Maryland).

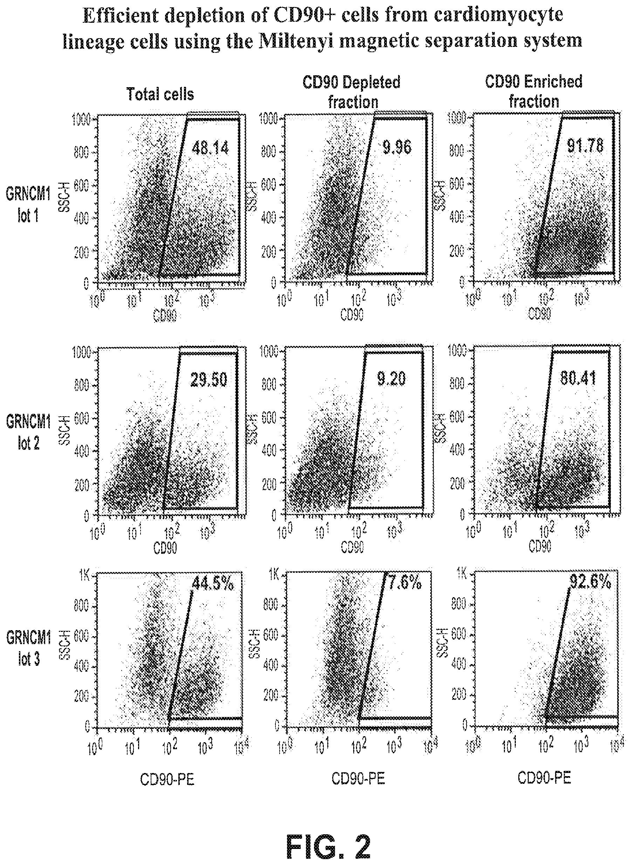

The efficiency of CD90 depletion of GRNCM1 was evaluated using flow cytometry FACSCalibur™ (Becton Dickinson, Franklin Lakes, NJ)Samples from each condition, (Total cells i.e. pre-depletion, CD90 depleted fraction, and CD90 enriched fraction) were evaluated for the presence of bound anti-human CD90 PE. Figure 2 shows the depletion efficiency for three different cardiomyocyte lineage lots. Comparing the total cells (pre-depletion) to the CD90 depleted fractions, the percentage of CD90 positive cells was reduced for each of the three lots tested. The enrichment of CD90 positive cells was observed to be > 80% in all three lots tested. The data demonstrate using the Miltenyi system for CD90 cell depletion is an efficient process for removing CD90+ cells from cardiomyocyte lineage preparations from hES cells.

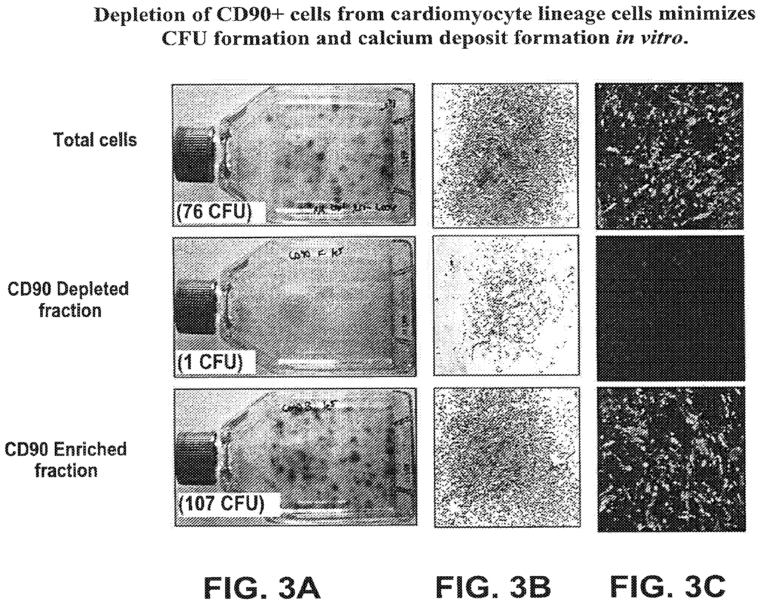

To determine if depletion of CD90+ cells correlated with depletion of MSCs cells from each of the conditions (Total cells pre-depletion, CD90 depleted fraction, and CD90 enriched fraction) were transferred to T25 flasks (Corning Life Sciences, Corning, NY) containing MSC maintenance medium (Lonza, Walkersville, Maryland) at 1e⁵ cells per flask. Cells were cultured for 14 days at 37°C 5% CO₂. Medium was exchanged and replaced every 4-5 days. After 14 days, cells in the T25 flasks were fixed in ice-cold 100% methanol for 5 minutes and rinsed 1X in 1X PBS. Cells were stained for 30 minutes with 0.5% crystal-violet (Sigma Aldrich, St. Louis, MO) dissolved in 100% methanol. Flasks were washed 2X in water and stained cells were air dried overnight. Colony forming units (CFUs) representing MSCs were counted visually under a light microscope. The cells in the CFU typically had a fibroblast-like morphology. The results are shown in Figure 3A & B. The CD90+ cell depletion reduced the number of CFU from 76 to 1 suggesting efficient depletion of colonies forming activity associated with MSCs.

The capacity to differentiate into osteoblasts was also evaluated for each of the three conditions tested for CFU activity. After 14 days in MSC maintenance medium (Lonza, Walkersville, Maryland), cells were subjected to osteoblasts differentiation medium (Lonza, Walkersville, Maryland) for an additional 14 days and the OsteoImage assay (Lonza, Walkersville, Maryland) was used to detect the presence of calcium deposits as described in example I. Calcium deposits were only found in the total cells and the enriched CD90+ fraction (Figure 3C). No calcium deposits were found in the CD90 depleted population indicating MSCs were efficiently removed.

### Reference Example III. Depletion of CD90 positive cells using direct isolation depletes MSCs from cardiomyocyte lineage cells differentiated in vitro from reference hES cells

CD90 positive cells were depleted from a preparation of cardiomyocyte lineage cells differentiated in vitro from an established line of human embryonic stem cells using the DynaBead® (Invitrogen, Carlsbad, CA) magnetic separation system and removal of MSCs was evaluated. Following the manufacturer's protocol, the direct cell isolation method was utilized. Unconjugated Mouse anti-CD90 antibody at 0.5µg (Biolgend, San Diego, CA) was added to 25µL (1e⁷ beads) of DynaBead® Pan Mouse IgG (Invitrogen, Carlsbad, CA), and incubated for 30 minutes at room temperature with gentle mixing. The tube containing the antibody mixture was placed in a magnet (Invitrogen, Carlsbad, CA) for 1 minute, supernatant was discarded, and the bead mixture was washed 2X with depletion buffer (0.1% BSA + 1X PBS). The bead mixture was resuspended in depletion buffer at the initial volume of DynaBeads Pan Mouse IgG (25µL/1e⁷ beads). The bead mixture was added to a preparation of cardiomyocyte lineage cells differentiated in vitro from a line of hES cells, at 15 beads per target cell, and incubated for 30 minutes at room temperature with gentle mixing. The tube containing the mixture of cells and beads was placed in a magnet for 2 minutes, and the supernatant containing the CD90 depleted cell population was transferred into a new tube.

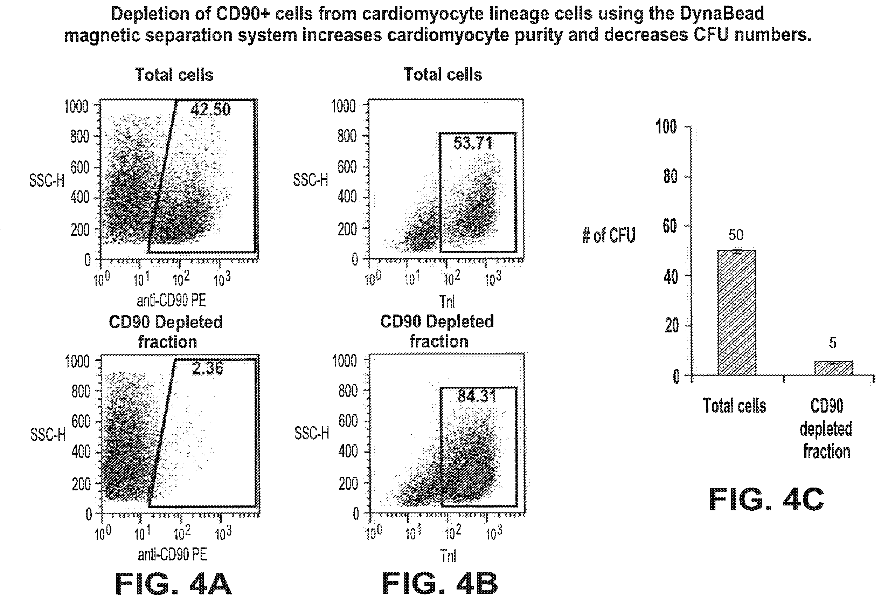

The efficiency of CD90 depletion of the cardiomyocyte lineage cells was evaluated using flow cytometry, FACSCalibur™ (Becton Dickinson, Franklin Lakes, NJ). Mouse Anti-CD90 antibody PE conjugated (IgG1) (Biolegend, San Diego, CA) (10µl/sample) was added to the total cells (i.e. an aliquot of pre-depleted cells) and CD90 depleted fraction. The comparison between the total cells and CD90 depleted fractions demonstrated the % of CD90 positive cells reduced after depletion (Figure 4A). The data suggests using the DynaBead system for CD90 cell depletion is an effective depletion method.

To determine if the depletion of CD90+ cells reduces the percentage of cardiomyocyte lineage cells in the preparation, the purity of cardiomyocyte lineage cells was also evaluated post depletion. Total cells (i.e. an aliquot of pre-depleted cells) and CD90 depleted fractions were fixed in 4% paraformaldehyde (Sigma Aldrich, St. Louis, MO) and permeabilized with 1X BD perm/wash buffer (BD Biosciences, San Jose, CA). Troponin I (TnI), a marker expressed by cardiomyocytes was detected using an anti-human TnI antibody (Millipore, Billerica, MA). Cells were incubated with anti-TnI (0.5µg/mL) antibody for 30 minutes at room temperature. Cells were washed 1X in 1X BD perm/wash buffer, and secondary anti-mouse IgG (H+L) AlexaA488 (Invitrogen, Carlsbad, CA) was added at 1:1000 dilution for 30 minutes. Cells were washed 1X in 1X BD perm/wash buffer, and the level of TnI positive cells was detected using flow cytometry. The results demonstrate CD90+ cell depletion from cardiomyocyte lineage preparation increases the % of cardiomyocyte lineage cells from 54% to 84% (Figure 4B). The data suggests CD90+ cells are primarily present on the non-cardiomyocyte population.

To evaluate the depletion efficiency of MSCs from GRNCM1, the enumeration of CFUs were determined as described in Example II. The total cells and CD90 depleted fractions were transferred to T75 flasks (Corning, Corning, NY) containing MSC medium (1.5e⁶ cells/flask)(Lonza, Walkersville, MD). After 14 days in culture, cells were fixed in methanol, and stained with 0.5% crystal violet. CFUs were enumerated visually under a light microscope. The data shows using the CD90+ depleted fractions had a 10 fold reduction in CFUs compared to the starting material (non-depleted fractions) (Figure 4C).

### Reference Example IV. Cell sorting of CD90 positive cells depletes MSCs from a preparation of cardiomyocyte lineage cells

Cardiomyocyte lineage cells differentiated in vitro from a line of reference hES cells were stained with mouse anti-CD90 PE (Biolegend, San Diego, CA) at 10µl per 1e⁶ cells at a final concentration of 1e7 cells/mL in staining buffer consisting of RPMI1640 (Invitrogen, Carlsbad, CA) + B27 (Invitrogen, Carlsbad, CA) + 1X penicillin/streptomycin (Invitrogen, Carlsbad, CA). CD90 positive and negative populations were sorted and collected into separate tubes using FACS Aria (BD Biosciences, San Jose, CA).

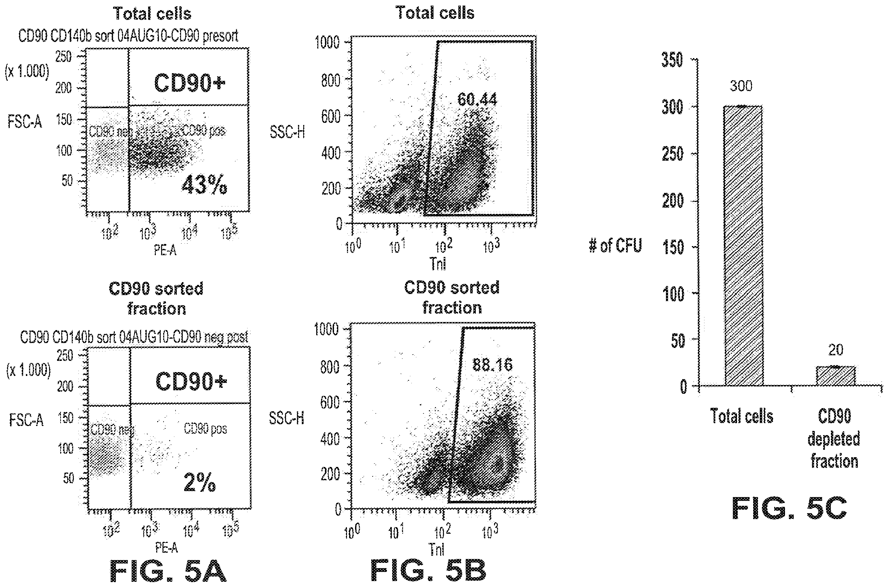

The efficiency of CD90 depletion was measured by the presence of bound anti-CD90 PE antibody in total cells (i.e. the pre-sorted population) and CD90 negative sorted cells using flow cytometry. Figure 5A shows the % of CD90 positive cells are reduced more than 20 fold in the CD90 sorted populations compared to the total cells. Intracellular staining to detect TnI+ cardiomyocyte lineage cells was also performed as described in Example III. Sorting out the CD90+ cells from cardiomyocyte lineage cells increased the % of cardiomyocyte lineage cells from the total cells from 60% to 88%, Figure 5B. CFU capability was also evaluated from total cells and CD90 sorted cells using methods described in Example II. Sorting CD90+ cells from cardiomyocyte lineage cells effectively eliminated the presence of MSCs (as measured by CFU capability) compared to the total cells (Figure 5C). Taken together, these data suggest cell sorting methods deplete CD90 from cardiomyocyte lineage cells differentiated in vitro from hES cells effectively removes MSCs while increasing cardiomyocytes purity.

### Reference Example V: Expression of MSC-associated cell surface markers in cardiomyocyte lineage cells

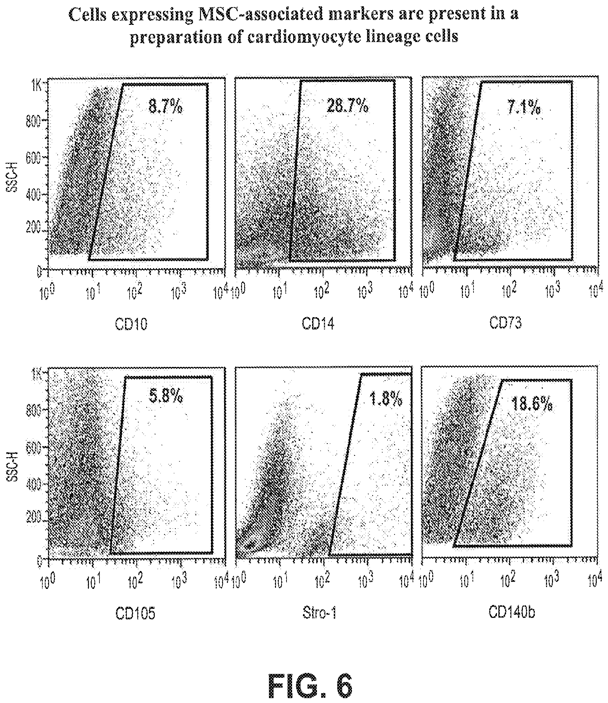

To identify MSCs within a population of cardiomyocyte lineage cells differentiated in vitro from an established line of reference hES cells, antibodies against the following markers were evaluated: CD10, CD73, CD105, CD44, CD140b, and Stro-1. Cardiomyocyte lineage cells were stained for 30 minutes with mouse anti-CDIO FITC conjugated, 10µl/test (BD Biosciences, San Jose, CA), anti-CD73 PE conjugated, 10µl/test, (BD Biosciences, San Jose, CA), anti-CD105 APC conjugated, 0.4µg/test, (Biolegend, San Diego, CA), anti-CD44 APC conjugated, 10µl/test, (BD Biosciences, San Jose, CA), anti-CD140b PE conjugated, 10µl/test, (BD Biosciences, San Jose, CA), and anti-Strol unconjugated, 0.5µg/test, (Santa Cruz Biotech, Santa Cruz, CA). Cells were washed 2X in 1% BSA 1X PBS. Cells stained with unconjugated antibodies were subsequently stained with a secondary detection antibody. Anti-mouse IgM A488 (Invitrogen, Carlsbad, CA) at 1:1000 dilution was added to detect anti-Strol. All samples were collected and analyzed on the FACSCalibur™ (BD Biosciences, San Jose, CA). Figure 6 shows flow cytometric dot plots for the indicated MSC-associated markers. The data supports the expression of MSC-associated markers in cardiomyocyte lineage cells.

### Reference Example VI: Depletion of CD10, CD73, CD105, or CD140b positive cells using indirect isolation depletes MSCs from cardiomyocyte lineage cells differentiated in vitro from reference hES cells

CD10, CD73, CD105, and CD140b markers expressed by MSCs, were used as individual target antigens for depletion of MSCs from cardiomyocyte lineage cells differentiated in vitro from reference hES cells. To deplete cells positive for these surface MSC markers from the cardiomyocyte lineage cells, the Miltenyi microbead system was utilized (Miltenyi Biotec, Auburn, CA). The cardiomyocyte lineage cells were resuspended in depletion buffer (PBS + 0.5% BSA + 2 mM EDTA) and mouse anti-human MSC marker antibody conjugated to PE against the described markers, (10µl/1e⁶ cells) (BD Biosciences, San Jose, CA) at a final cell concentration of 2.0-4.0e⁷ cells/mL. Cells were incubated with the antibody for 20 minutes at 4°C and then washed 1X and resuspended with depletion buffer at 1e⁷cells/80µL. Following the manufacturer's instructions, anti-PE micro beads (Miltenyi Biotec, Auburn, CA) were added to the cells at 20µL per 1e⁷ cells. Cells were incubated for 15 minutes at 4°C and then washed 1X and resuspended with depletion buffer using 500µL of buffer for cell numbers up to 1e⁸ cells. Cells were added to a MS depletion column (Miltenyi Biotec, Auburn, CA) that had previously been washed with 3mL of depletion buffer, and then attached to a MidiMACS Separator (Miltenyi Biotec, Auburn, CA). Non-bound cells passed through the column and were collected in a sterile 15mL polypropylene tube (Corning Life Sciences, Corning, NY). The column was washed 3X with 3mL of depletion medium. Each successive wash was applied to the column once the column was empty. The MS depletion column was removed from the separator and 4mL of depletion buffer was added to the column. To isolate cells bound to the column, a plunger was used to flush out the cells into a sterile 15mL polypropylene tube. Cells were washed 2X with depletion buffer, and resuspended in MSC maintenance medium (Lonza, Walkersville, Maryland).

The efficiency of MSC-associated marker depletion from cardiomyocyte lineage cells was evaluated using flow cytometer FACSCaliburTM (BD Biosciences, San Jose, CA). Samples from each condition, (Total cells i.e., pre-depletion, MSC marker depleted fraction, and MSC marker enriched fraction) were evaluated for the presence of bound anti-human MSC marker conjugated to PE. Figure 6A-D shows the depletion efficiency of CD10, CD73, CD105, and CD140b from cardiomyocyte lineage cells. Comparing the total cells (pre-depletion) to the MSC marker depleted fractions, the percentage of MSC marker positive cells were all reduced. The enrichment of MSC marker positive cells were observed to be > 50% from the cardiomyocyte lineage cells tested. The data suggests using the Miltenyi system for MSC marker cell depletion can reduce the percentage of MSC marker positive cells in the cardiomyocyte lineage preparations from hES cells.

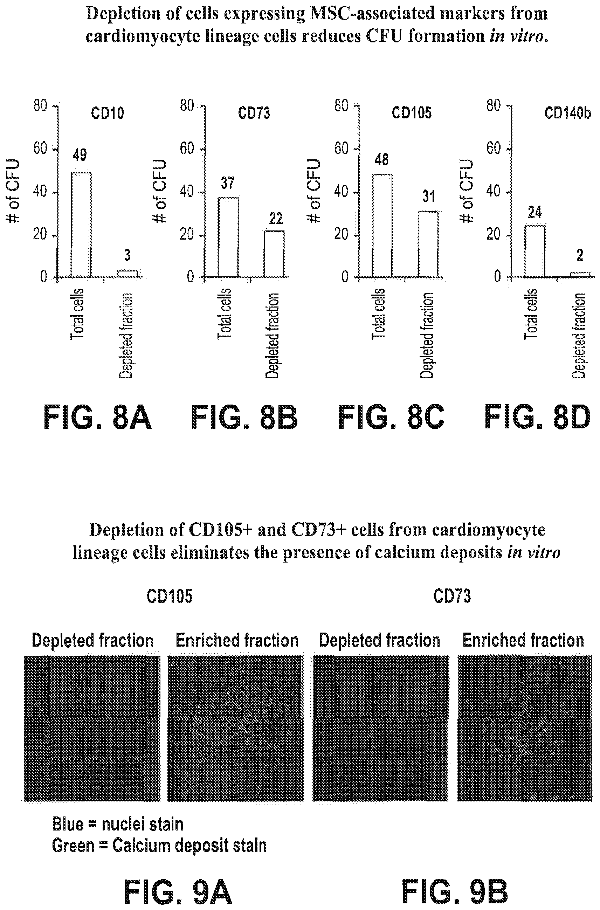

To determine if depletion of MSC marker positive cells (CD10, CD73, CD105, and CD140b) correlated with depletion of MSCs cells from each of the conditions (Total cells pre-depletion, MSC-marker depleted fraction, and MSC-marker enriched fraction) cells were transferred to T25 flasks (Corning Life Sciences, Corning, NY) containing MSC maintenance medium (Lonza, Walkersville, Maryland) at 2.5-1.0e⁵ cells per flask. Cells were cultured for 14 days at 37°C 5% CO₂. Medium was exchanged and replaced every 4-5 days. After 14 days, cells in the T25 flasks were fixed in ice-cold 100% methanol for 5 minutes and rinsed once in 1X PBS. Cells were stained for 30 minutes with 0.5% crystal-violet (Sigma Aldrich, St Louis, MO) dissolved in 100% methanol. Flasks were washed 2X in water and stained cells were air dried overnight. Colony forming units (CFUs) representing MSCs were counted visually under a light microscope. The results are shown in Figure 8A-D. Targeting MSC-marker positive cells for depletion reduced the number of CFU compared to pre-depleted cells, suggesting a depletion effect of colony forming activity associated with MSCs.

The capacity to differentiate into osteoblasts was evaluated using the depleted and enriched fractions of cells expressing MSC-associated marker CD73 and CD105 from the magnetic separation. After 14 days in MSC maintenance medium (Lonza, Walkersville, Maryland), cells were incubated with osteoblasts differentiation medium (Lonza, Walkersville, Maryland) for an additional 14 days and the OsteoImage assay (Lonza, Walkersville, Maryland) was used to detect the presence of calcium deposits. Calcium deposits were primarily found in fraction containing the cells enriched for the MSC-associated markers, Figure 9A-B. No calcium deposits were found in the MSC-marker depleted population indicating MSCs were efficiently removed.

### Reference Example VII: Cell sorting of CD140b and CD10 positive cells depletes MSCs from a preparation of cardiomyocyte lineage cells.

Cardiomyocyte lineage cells differentiated in vitro from a line of reference hES cells were stained with mouse anti-CD140b PE (BD Biosciences, San Jose, CA) and mouse anti-CD10 PE (BD Biosciences, San Jose, CA) at 10µl per 1e⁶ cells at a final concentration of 1e⁷ cells/mL in staining buffer containing RPMI1640 (Invitrogen, Carlsbad, CA) + B27 (Invitrogen, Carlsbad, CA) + 1X penicillin/streptomycin (Invitrogen, Carlsbad, CA). CD140b and CD10 +/populations were sorted and collected into separate tubes using the FACS Aria (BD Biosciences, San Jose, CA).

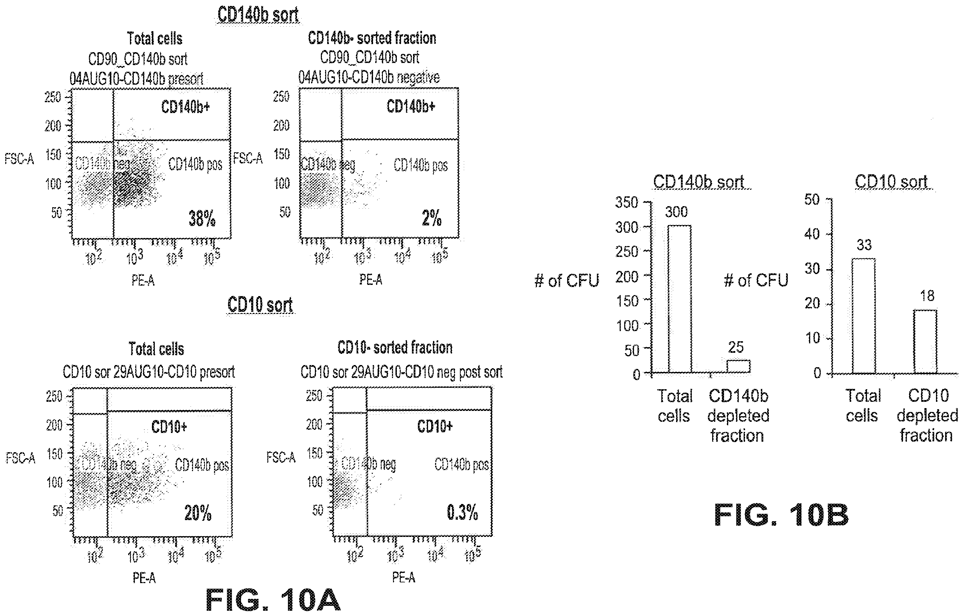

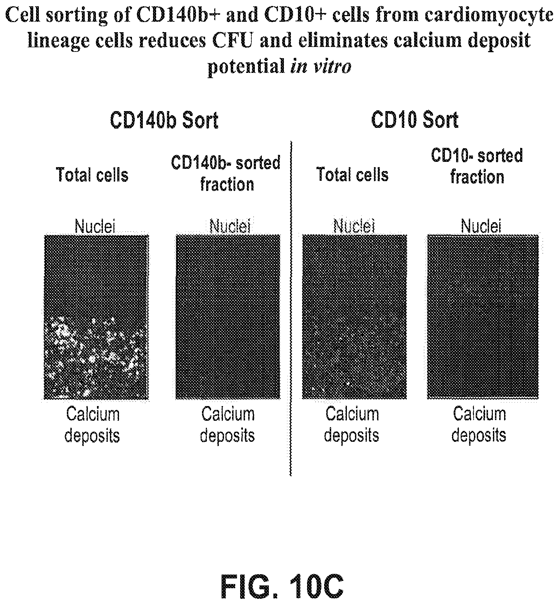

The efficiency of CD140b and CD10 depletion was measured by the presence of bound anti-CD140b and anti-CD10 PE antibody in total cells (pre-depleted) and negative sorted cells using flow cytometry. Figure 10A shows the % of CD140b and CD10 positive cells are reduced in the CD140b and CD10 sorted negative populations compared to the total cells. CFU capability was also evaluated from total cells and CD140b negative, and CD10 negative sorted cells. Sorting the CD140b and CD10 negative cells from cardiomyocyte lineage cells reduced the presence of MSCs (as measured by CFU capability) compared to the total cells (Figure 10B) and removed osteoblasts potential by the absence of calcium deposits (Figure 10C). Taken together, these data suggest cell sorting methods that deplete CD140b and CD10 positive cells from cardiomyocyte lineage cells differentiated in vitro from hES cells effectively removed MSCs.

### Reference Example VIII: CD106 Is a Marker for Cardiomyocyte Lineage Cells

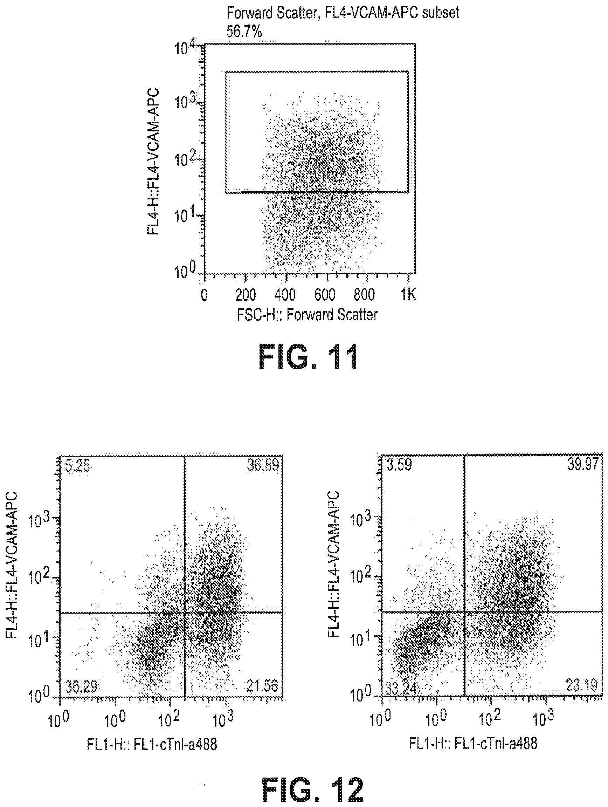

CD106 (VCAM-1), a cell adhesion molecule, was used as a target antigen for cardiomyocyte identification from cardiomyocyte lineage cells differentiated in vitro from reference hES cells (CM's). To label CM's, CD106 (clone IE10, R&D Systems, Minneapolis, MN) antibody was evaluated. The cardiomyocyte lineage cells were resuspended in Flow Cytometry (FC) buffer (PBS + 2% heat-inactivated Fetal Bovine Serum + 0.09% NaN3) and mouse anti-human CD106 antibody conjugated to APC at a final cell concentration of 0.1ug/5e⁵ cells/100uL. Cells were incubated with the antibody for 30 minutes at 4°C. The cells were washed and resuspended for analysis in FC buffer containing 1 ug/ml of propidium iodide (Sigma) to identify nonviable cells. Stained samples were examined using flow cytometry (FACSCalibur™, Becton Dickinson, Franklin Lakes, NJ,) for the presence of bound anti-human CD106 (Figure 11). To determine if CD106 expressing cells correlate with cardiomyocyte lineage cells, cells (in vitro differentiated progeny from a line of hES cells) were co-labeled with known cardiomyocyte markers. Cardiomyocyte markers β-actinin (sarcomeric) (clone AE-53, Sigma, St Louis, MO) and cardiac troponin I (clone 8E10, Millipore, Billerica, MA), were used for co-labeling CM's with CD106. CM's that were stained with CD106 were then tagged with 5ug/mL Ethidium Monoazide (EMA) (Sigma, St Louis, MO) and to identify nonviable cells. Cells were placed on ice and exposed to bright light for 10 minutes to crosslink EMA to DNA of dead cells. Cells were fixed for 10 minutes in 2% paraformaldehyde and then permeabilized with BD Perm/Wash™ (Becton Dickinson, Franklin Lakes, NJ) for 15 minutes at room temperature. Cells were labeled with β-actinin (0.5ug/5e⁵ cells/100uL) or cTnI (0.1ug/5e⁵ cells/100uL) antibodies for 30 minutes at 4C in BD Perm/Wash™. Cells were then washed and incubated in BD Perm/Wash™ with the addition of Alexa Fluor 488-conjugated anti-mouse secondary antibody (Invitrogen, Carlsbad, CA) for 30 minutes at 4C. Cells were washed 1x in BD Perm/Wash™ and resuspended in 200 uL FC buffer. The efficiency of co-labeling CD 106 with known cardiomyocyte markers were evaluated using flow cytometry (Figure 12). The data demonstrate that CD106 is a marker for labeling cardiomyocytes generated from hES cells. The marker may be useful in evaluating cardiomyocyte lineage cell populations depleted of extraneous phenotypic cells as described infra.

### Reference Example IX: The Majority of CD106+ Cells are CD90-

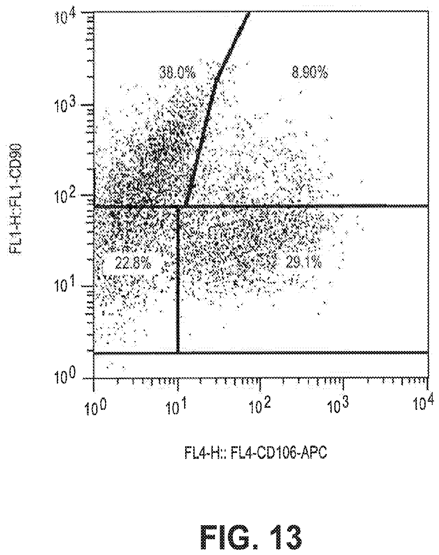

CD106 (VCAM-1), a cell adhesion molecule, has been evaluated as a target antigen for cardiomyocyte identification from cardiomyocyte lineage cells differentiated in vitro from hES cells (CM's). A surface marker screen was performed evaluating CD106 expression along with BD Lyoplate Human Cell Surface Marker Panel (Becton Dickinson, Franklin Lakes, NJ). The cardiomyocyte lineage cells were resuspended in Flow Cytometry (FC) buffer (PBS + 2% heat-inactivated Fetal Bovine Serum + 5 mM EDTA) and filtered through a 70 uM cell strainer (Becton Dickinson, Franklin Lakes, NJ) to remove clumps. Cells were labeled with CD90 (clone 5E10, Becton Dickinson, Franklin Lakes, NJ) at 0.5ug/5e⁵ cells/100uL for 30 minutes at 4C. The CM's were then washed and incubated in FC buffer with the addition of Alexa Fluor 488-conjugated anti-mouse secondary antibody (Invitrogen) for 30 minutes at 4C. Cells were then washed and incubated in FC buffer with the addition of mouse anti-human CD106 antibody conjugated to APC (clone IE10, R&D Systems, Minneapolis MN) at a final cell concentration of 0.1ug/5e⁵ cells/100uL. The cells were washed and resuspended for analysis in FC buffer containing lug/ml of propidium iodide (Sigma) to identify nonviable cells. Stained samples were examined using flow cytometry FACSCalibur™ (Becton Dickinson, Franklin Lakes, NJ) for expression of markers (Figure 13).

The results demonstrated that the majority of CD90+ cells were negative for CD106 and the majority of CD106+ cells were negative for CD90. Thus targeting CD90+ cells for depletion will enrich for CD106+ cells such as cardiomyocyte lineage cells.

### Reference Example X: Depletion of CD90 Positive Cells Using Indirect Isolation Enriches for CD106+ Cells from Cardiomyocyte Lineage Cells Differentiated in vitro From reference hES Cells

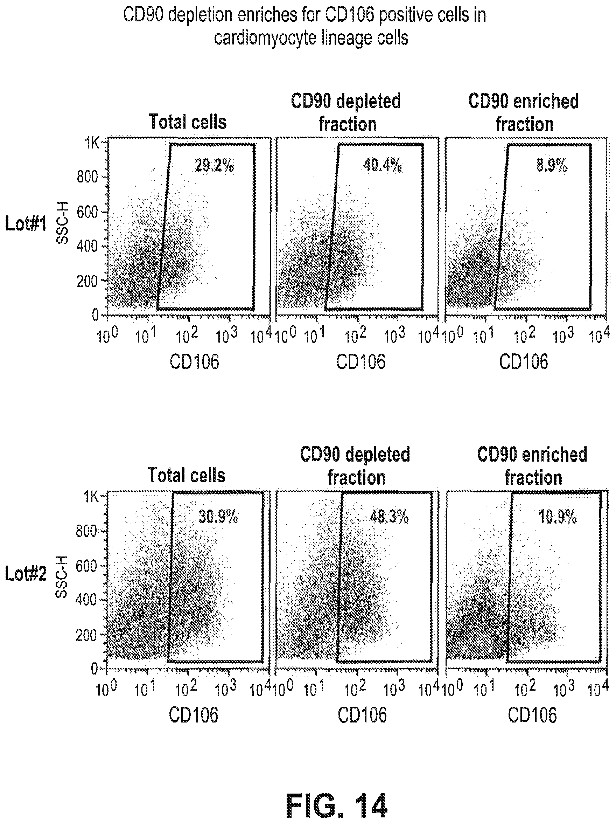

To deplete CD90+ cells from the cardiomyocyte lineage cells, the Miltenyi microbead system was utilized (Miltenyi Biotec, Auburn, CA) as described previously. CD106 expression was evaluated using flow cytometry FACSCalibur™ (Becton Dickinson, Franklin Lakes, NJ). Samples from each condition, (Total cells i.e. pre-depletion, CD90 depleted fraction, and CD90 enriched fraction) were stained for 30 minutes with anti-human CD106 conjugated to APC (10µl/test)(BD Biosciences, San Jose, CA). The cells were washed two times with 1%BSA/1x PBS and then evaluated by flow cytometry. Figure 14 shows the expression of CD106 for two different cardiomyocyte lineage lots. Comparing the total cells (pre-depletion) to the CD90 depleted fractions, the percentage of CD106 positive cells was increased. The data demonstrates using the Miltenyi system for CD90 cell depletion is an efficient process for enriching cardiomyocyte lineage preparations from hES cells.

## Claims

1. A method of reducing the number of mesenchymal stem cells (MSC) in a mixed population of cells comprising cardiomyocyte lineage cells which are the in vitro differentiated progeny of primate pluripotent stem (pPS cells), the method comprising: a) contacting the mixed cell population with one or more ligands that bind to a MSC marker chosen from CD90, CD73, CD140b, and CD10; and b) removing the ligand bound cells of a) thereby reducing the number of MSC from the mixed population of cells comprising cardiomyocyte lineage cells.

2. The method of claim 1, wherein the one or more ligands that bind to a MSC marker chosen from CD90, CD73, CD140b, and CD10 is one or more antibodies.

3. The method of claim 2, wherein the one or more antibodies is a monoclonal antibody.

4. The method of claim 2, wherein the one or more antibodies is a polyclonal antibody.

5. The method of claim 2, wherein the one or more antibodies is an antibody that binds to CD90.

6. The method of claim 1, wherein the removing the ligand bound cells comprises contacting the cell population of a) with an external force.

7. The method of claim 6, wherein the external force is a magnetic field.

8. The method of claim 1, wherein the ligand is bound to a solid support.

9. The method of claim 8, wherein the ligand is directly bound to the solid support.

10. The method of claim 8, wherein the ligand is indirectly bound to the solid support.

11. The method of claim 8, wherein the solid support is a bead.

12. The method of claim 11, wherein the bead is a magnetic bead.

13. The method of claim 1, wherein the cardiomyocyte lineage cells express one or more markers chosen from CD106, cardiac troponin I (cTnI), cardiac troponin T (cTnT), Nkx2.5, ANF, myosin heavy chain (MHC), titin, tropomyosin, α sarcomeric actinin, desmin, GATA-4, MEF 2A, MEF 2B, MEF 2C, MEF 2D, N-cadherin, connexin 43, β1 adrenoreceptor (β1 AR), creatine kinase MB (CK MB), myoglobin, and α cardiac actin.

14. The method of claim 1, wherein the obtaining step comprises differentiating in vitro an aliquot of a line of pPS cells into cardiomyocyte lineage cells.

15. The method of claim 1, wherein the ligand is conjugated to a detectable substance.

16. The method of claim 15, wherein the detectable substance is a dye.

17. The method of claim 1, wherein removing the ligand bound cells comprises sorting the ligand bound cells using a flow cytometer.

## Patentansprüche

1. Verfahren zum Reduzieren der Anzahl mesenchymaler Stammzellen (MSC) in einer gemischten Population von Zellen, die Kardiomyozyten-Abstammungszellen umfassen, bei denen es sich um in vitro differenzierte Nachkommen pluripotenter Primaten-Stammzellen (pPS-Zellen) handelt, wobei das Verfahren umfasst:
a) Inkontaktbringen der gemischten Zellpopulation mit einem oder mehr Liganden, die an einen MSC-Marker, ausgewählt aus CD90, CD73, CD140b und CD10, binden; und
b) Entfernen der ligandengebundenen Zellen von a), wodurch die Anzahl von MSC aus der gemischten Population von Zellen, die Kardiomyozyten-Abstammungszellen umfassen, reduziert wird.

2. Verfahren nach Anspruch 1, wobei es sich bei den ein oder mehr Liganden, die an einen MSC-Marker, ausgewählt aus CD90, CD73, CD140b und CD10, binden, um einen oder mehr Antikörper handelt.

3. Verfahren nach Anspruch 2, wobei es sich bei den ein oder mehr Antikörpern um einen monoklonalen Antikörper handelt.

4. Verfahren nach Anspruch 2, wobei es sich bei den ein oder mehr Antikörpern um einen polyklonalen Antikörper handelt.

5. Verfahren nach Anspruch 2, wobei es sich bei den ein oder mehr Antikörpern um einen Antikörper handelt, der an CD90 bindet.

6. Verfahren nach Anspruch 1, wobei das Entfernen der ligandengebundenen Zellen das Inkontaktbringen der Zellpopulation von a) mit einer externen Kraft handelt.

7. Verfahren nach Anspruch 6, wobei es sich bei der externen Kraft um ein Magnetfeld handelt.

8. Verfahren nach Anspruch 1, wobei der Ligand an einen festen Träger gebunden ist.

9. Verfahren nach Anspruch 8, wobei der Ligand direkt an den festen Träger gebunden ist.

10. Verfahren nach Anspruch 8, wobei der Ligand indirekt an den festen Träger gebunden ist.

11. Verfahren nach Anspruch 8, wobei es sich bei dem festen Träger um ein Kügelchen handelt.

12. Verfahren nach Anspruch 11, wobei es sich bei dem Kügelchen um ein magnetisches Kügelchen handelt.

13. Verfahren nach Anspruch 1, wobei die Kardiomyozyten-Abstammungszellen einen oder mehr Marker exprimieren, ausgewählt aus CD106, Herztroponin I (cTnI), Herztroponin T (cTnT), Nkx2.5, ANF, schwerer Myosinkette (MHC), Titin, Tropomyosin, einem sarkomeren Aktinin, Desmin, GATA-4, MEF 2A, MEF 2B, MEF 2C, MEF 2D, N-Cadherin, Connexin 43, β1-Adrenozeptor (β1-AR), Kreatinkinase MB (CK MB), Myoglobin, und α-Herzaktin.

14. Verfahren nach Anspruch 1, wobei der Erhaltungsschritt das In-vitro-Differenzieren eines Aliquots einer Linie von pPs-Zellen in Kardiomyozyten-Abstammungszellen umfasst.

15. Verfahren nach Anspruch 1, wobei der Ligand an eine nachweisbare Substanz konjugiert ist.

16. Verfahren nach Anspruch 15, wobei es sich bei der nachweisbaren Substanz um einen Farbstoff handelt.

17. Verfahren nach Anspruch 1, wobei das Entfernen der ligandengebundenen Zellen das Sortieren der ligandengebundenen Zellen mit einem Durchflusszytometer umfasst.

## Revendications

1. Procédé de réduction du nombre de cellules souches mésenchymateuses (CSM) dans une population mélangée de cellules comprenant des cellules d'une lignée de cardiomyocytes, qui sont la descendance différenciée in vitro de cellules souches pluripotentes de primate (cellules pPS), le procédé comprenant les étapes consistant à : a) mettre en contact la population mélangée de cellules avec un ou plusieurs ligand(s) qui se lie/lient à un marqueur des CSM choisi parmi les CD90, CD73, CD140b et CD10 ; et b) ôter les cellules liées au ligand du paragraphe a), en réduisant de ce fait le nombre de CSM à partir de la population mélangée de cellules comprenant des cellules d'une lignée de cardiomyocytes.

2. Procédé de la revendication 1, dans lequel l'un ou les plusieurs ligand(s) qui se lie/lient à un marqueur des CSM, choisi parmi les CD90, CD73, CD140b et CD10, est/sont un ou plusieurs anticorps.

3. Procédé de la revendication 2, dans lequel l'un ou les plusieurs anticorps est/sont un anticorps monoclonal.

4. Procédé de la revendication 2, dans lequel l'un ou les plusieurs anticorps est/sont un anticorps polyclonal.

5. Procédé de la revendication 2, dans lequel l'un ou les plusieurs anticorps est/sont un anticorps qui se lie au CD90.

6. Procédé de la revendication 1, dans lequel le retrait des cellules liées au ligand comprend une mise en contact de la population de cellules du paragraphe a) avec une force externe.

7. Procédé de la revendication 6, dans lequel la force externe est un champ magnétique.

8. Procédé de la revendication 1, dans lequel le ligand est lié à un support solide.

9. Procédé de la revendication 8, dans lequel le ligand est directement lié au support solide.

10. Procédé de la revendication 8, dans lequel le ligand est indirectement lié au support solide.

11. Procédé de la revendication 8, dans lequel le support solide est une bille.

12. Procédé de la revendication 11, dans lequel la bille est une bille magnétique.

13. Procédé de la revendication 1, dans lequel les cellules de la lignée de cardiomyocytes expriment un ou plusieurs marqueur(s) choisi(s) parmi le CD106, la troponine I cardiaque (TnIc), la troponine T cardiaque (TnTc), le Nkx2.5, l'ANF, une chaîne lourde de myosine (CLM), la titine, la tropomyosine, l'actinine sarcomérique a, la desmine, les GATA-4, MEF 2A, MEF 2B, MEF 2C, MEF 2D, la N-cadhérine, la connexine 43, un récepteur adrénergique β1 (AR β1), la créatine kinase MB (CK MB), la myoglobine et l'actine cardiaque a.

14. Procédé de la revendication 1, dans lequel l'étape d'obtention comprend une différenciation in vitro d'une aliquote d'une lignée de cellules pPS en cellules d'une lignée de cardiomyocytes.

15. Procédé de la revendication 1, dans lequel le ligand est conjugué à une substance détectable.

16. Procédé de la revendication 15, dans lequel la substance détectable est un colorant.

17. Procédé de la revendication 1, dans lequel le retrait des cellules liées au ligand comprend un tri des cellules liées au ligand en utilisant un cytomètre en flux.
